(19) **Europäisches Patentamt**
**European Patent Office**
**Office européen des brevets**

(11) **EP 4 775 219 A1**

(12) **EUROPEAN PATENT APPLICATION**
published in accordance with Art. 153(4) EPC

(43) Date of publication:
**15.07.2026 Bulletin 2026/29**

(21) Application number: **24862005.6**

(22) Date of filing: **04.09.2024**

(51) International Patent Classification (IPC):
*A61K 38/48* (2006.01)  *C12N 15/57* (2006.01)
*C12N 15/85* (2006.01)  *C12N 15/86* (2006.01)
*A61P 35/00* (2006.01)

(52) Cooperative Patent Classification (CPC):
**A61K 38/48; A61P 35/00; C12N 9/48; C12N 15/85;**
**C12N 15/86;** Y02A 50/30

(86) International application number:
**PCT/CN2024/116936**

(87) International publication number:
**WO 2025/051164 (13.03.2025 Gazette 2025/11)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **07.09.2023  CN 202311152705**

(71) Applicant: **Academy of Military Medical Sciences**
**Beijing 100850 (CN)**

(72) Inventors:
• **ZHONG, Wu**
**Beijing 100850 (CN)**

• **CAO, Ruiyuan**
**Beijing 100850 (CN)**
• **YANG, Xiaotong**
**Beijing 100850 (CN)**
• **LI, Wei**
**Beijing 100850 (CN)**
• **LI, Song**
**Beijing 100850 (CN)**

(74) Representative: **Lavoix**
**Bayerstraße 83**
**80335 München (DE)**

Remarks:
The complete document including Reference
Table(s) and the Sequence Listing(s) can be
downloaded from the EPO website

(54) **USE OF VIRUS 3C PROTEASE IN PREVENTION OR TREATMENT OF TUMOR**

(57)    Provided are a use of a virus 3C protease or a nucleic acid molecule encoding the 3C protease in the prevention and/or treatment of a tumor in a subject (e.g., a human), and a use of the 3C protease or the nucleic acid molecule encoding the 3C protease in the preparation of a drug for preventing and/or treating a tumor in a subject (e.g., a human).

**EP 4 775 219 A1**

## Description

The present application is based on and claims the benefit of priority from Chinese Application No. 202311152705.0, filed on September 7, 2023, the disclosures of which are incorporated herein by reference in its entirety.

## Technical Field

The present application relates to the field of viruses and the field of tumor treatment. Specifically, the present application relates to the use of viral 3C protease or a nucleic acid molecule encoding the 3C protease for the prevention and/or treatment of tumors in a subject (e.g., a human), and in the preparation of a medicament for the prevention and/or treatment of tumors in a subject (e.g., a human).

## Background Art

Cancer is a disease characterized by the uncontrolled division and proliferation of cells in the body. In the United States, approximately one-third of all women and half of all men will develop cancer in their lifetime. Cancers are generally classified into two categories: solid tumors and disseminated cancers. For many types of cancer, treatment regimens may include one or a combination of surgery, chemotherapy, radiotherapy, bone marrow/stem cell transplantation, anticancer drugs and immunotherapy. Although these treatments may be effective, they are typically associated with numerous adverse effects. Chemotherapy specifically targets all newly divided cells in the body, not just cancer cells. Gene therapy and genetic vaccination are among the most promising and rapidly developing approaches in modern medicine. They can offer highly specific and personalized options for the treatment of a wide variety of diseases.

Compared with DNA and viral vectors, mRNA does not be inserted into the genome, but only enables the transient expression of encoded proteins, thus it offers an excellent and safe option researchers and pharmaceutical companies due to its low insertion risk. mRNA exerts its functions without the need to enter the nucleus, and therefore carries no risk of stable integration into the host cell genome. Upon reaching the cytoplasm, mRNA initiates protein translation. mRNA is easily synthesized by the *in vitro* transcription (IVT) process. And mRNA encapsulated in lipid nanoparticles enables the targeted delivery to organs such as the liver and/or spleen.

The most common primary brain tumor in adults is glioblastoma, which is also one of the most lethal cancers. For glioblastoma, conventional treatment involves surgical resection followed by radiotherapy and concurrent chemotherapy. Even with this treatment regimen, the median survival of glioblastoma patients is less than 15 months. The poor prognosis is mainly attributed to tumor recurrence, which is believed to originate from a subset of cancer stem cells that survived primary treatment. Recent studies have shown that glioblastoma stem cells survive longer under treatment stress and become more aggressive upon recurrence, thereby developing resistance to primary chemotherapy. However, while chemotherapy drugs inhibit and kill tumors, they also have a killing effect on normal cells of the body, particularly on rapidly proliferating normal cells such as bone marrow hematopoietic cells and gastrointestinal mucosal epithelial cells. Therefore, the toxic and side effects of chemotherapy drugs have become the main obstacle to improving their therapeutic efficacy.

Currently, clinical research on brain gliomas mainly encompasses hormonal therapy, immunotherapy, oncolytic virotherapy and gene therapy. Endogenous ovarian steroid hormones are also involved in the progress of gliomas, and steroid hormones, especially endogenous estrogen, play a crucial role in brain development and differentiation. There is still a lot of controversy about the risk of gliomas and the side effects on the human body caused by long-term use of hormone replacement therapy. While immunotherapies such as targeting PD-1 and peptide-based vaccines have certain efficacy, the clinical application of immunotherapy for glioblastoma still faces several challenges, including the induction of secondary brain tumors and excessive activation of autoimmunity. Oncolytic virotherapy is an emerging novel therapeutic approach currently under investigation in the preclinical and clinical stages. Several viruses, including herpes simplex virus, adenovirus, poliovirus, and reovirus, are currently in Phase I and Phase II clinical trials for the treatment of glioblastoma and have been shown to improve overall survival. Most oncolytic viruses fail to overcome many obstacles, including being neutralized and cleared by antibodies, and there are also potential risks such as cytokine storm in the body induced by the introduction of a large number of viruses into the blood. To date, none have been approved by the FDA. mRNA therapy, a branch of gene therapy, has been explored as one of the safest and most effective therapeutic strategies for tumors. mRNA is composed of four nucleic acids, abbreviated as the letters A, U, C, and G. Cells decode the sequence of these nucleic acid arrangements much like the ingredients in a protein formula. If the mRNA formula cannot be decoded, the therapy exerts no effect and thus causes no side effects. If an opposite situation occurs, the body will only produce the proteins it needs at the right time and in the right place. Therefore, mRNA-based drug therapy represents a precise and non-toxic therapeutic approach for anti-cancer and antiviral treatments.

## Contents of the Disclosure

Based on extensive experimental studies, the inventors of the present application have unexpectedly found that 3C proteases derived from picornaviruses (e.g., *Enterovirus 71, Coxsackievirus A14*) has a broad-spectrum and significant tumor cell-killing activity. Based on this discovery, the inventors of the present application have developed a new method for the prevention and/or treatment of a tumor based on the 3C protease or a nucleic acid molecule encoding the 3C protease. The therapeutic drugs (e.g., nucleic acid drugs) provided in the present application, which are based on the 3C protease or the nucleic acid molecule encoding the 3C protease, are expected to expand the scope of benefits for cancer patients, improve the survival rate of patients, and bring more therapeutic benefits to the patients themselves, their families and the society, meanwhile, they also open up a brand-new avenue for the research of anti-tumor gene therapy drugs.

Therefore, in a first aspect, the present application provides a use of a 3C protease derived from a picornavirus or a nucleic acid molecule encoding the 3C protease in the preparation of a medicament for the prevention and/or treatment of a tumor in a subject.

In certain embodiments, the picornavirus is an enterovirus, such as a human enterovirus.

In certain embodiments, the picornavirus is selected from the group consisting of novel enterovirus, poliovirus, coxsackievirus, rhinovirus, and echovirus.

In certain embodiments, the picornavirus is a novel enterovirus (e.g., *Enterovirus 68* (EV68), *Enterovirus 69* (EV69), *Enterovirus 70* (EV70), *Enterovirus 71* (EV71)), a coxsackievirus (e.g., *Coxsackievirus A14, Coxsackievirus A16, Coxsackievirus A6, Coxsackievirus B3),* or a rhinovirus (e.g., *Rhinovirus 2, Rhinovirus 7, Rhinovirus 25).*

In certain embodiments, the picornavirus is *Enterovirus 71* (EV71) or *Coxsackievirus A14*.

In certain embodiments, the *Enterovirus 71* (EV71) is of genotype C4 or genotype or C5.

In certain embodiments, the *Enterovirus 71* (EV71) is a clinical isolate AH08/06.

In certain embodiments, the picornavirus is an aphthovirus.

In certain embodiments, the aphthovirus is a foot-and-mouth disease virus.

In certain embodiments, the foot-and-mouth disease virus is selected from the group consisting of European serotypes (Serotype A, Serotype O, Serotype C), Asian serotype (Asian Serotype 1), and African serotypes (Serotype SAT1, Serotype SAT2, Serotype SAT3).

In certain embodiments, the aphthovirus is selected from the group consisting of strain A10-61, strain HKN/2002, strain KEN/4/2013.

It is easy for those skilled in the art to understand that the "3C protease" described herein may be directly derived from the picornavirus (e.g., wild-type or mutant picornavirus), or alternatively, obtained by artificial modification (e.g., introduction of mutations or modifications) of the 3C protease directly derived from the picornavirus, provided that it retains the biological activity of the 3C protease from which it is derived (e.g., oncolytic activity). In addition, the 3C protease described herein may be a full-length 3C protease or an active fragment thereof (e.g., an active fragment having oncolytic activity).

In certain embodiments, the 3C protease is derived from a novel enterovirus (e.g., strain HZ08(Genbank: HQ400942.1), strain Mahoney (Genbank: V01149.1), strain CHN_HN_2019_214 (Genbank: ON755036.1)). In certain embodiments, the 3C protease is as set forth in any one of Uniprot: F5A4L9, P03300, and A0A075FJJ1.

In certain embodiments, the 3C protease is derived from a rhinovirus (e.g., strain HRV-1A_P25 (Genbank: KC894169.1), strain HRV-1A_P8 (Genbank: KC894168.1), strain RvA1B/KZ/2021/87 (Genbank: OP886969.1)). In certain embodiments, the 3C protease is as set forth in any one of Uniprot: P23008, A0A0F6VY48, and P12916.

In certain embodiments, the 3C protease is derived from an aphthovirus (e.g., strain A10-61 (Genbank: M31575.1), strain HKN/2002 (Genbank: AY317098.1), strain KEN/4/2013 (Genbank: OM863583.1)). In certain embodiments, the 3C protease is as set forth in any one of Uniprot: P03306, AY317098.1, and F1AW55.

It is easy for those skilled in the art to understand that the 3C protease may further contain an additional polypeptide to facilitate the expression, purification and/or tracing of the protein.

In certain embodiments, the additional polypeptide is optionally linked to the N-terminus or C-terminus of the 3C protease via a linker (e.g., a peptide linker). Such linkers (e.g., peptide linkers) are well known in the art, and examples of which include, but are not limited to, peptide linkers comprising one or more (e.g., 1, 2, 3, 4 or 5) amino acids (e.g., Gly or Ser). In certain embodiments, the peptide linker is flexible. In certain embodiments, a flexible peptide linker may be advantageous, as it is capable of linking the two protein/polypeptide components while maintaining their respective activities and functions. Such peptide linkers include, but are not limited to, (GGGGS)$_n$.

In certain embodiments, the additional polypeptide is selected from the group consisting of tag, signal peptide or leader peptide, detectable label (e.g., luciferase (such as fluc), green fluorescent protein (GFP)), and any combination thereof.

In certain embodiments, the 3C protease has an amino acid sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the amino acid sequence as set forth in SEQ ID NO: 1.

In certain embodiments, the 3C protease has an amino acid sequence as set forth in SEQ ID NO: 1. The sequence shown herein does not comprise an amino acid (e.g., methionine (Met)) encoded by an initiation codon (e.g., ATG) at its N-terminus. Those skilled in the art understand that, in the process of preparing proteins by genetic engineering, due to the function of the initiation codon, the first amino acid of the resulting polypeptide chain is often the amino acid (e.g., Met) encoded by the initiation codon. The 3C protease of the present application not only comprises amino acid sequences that do not contain an amino acid (e.g., Met) encoded by an initiation codon at its N-terminus, but also comprises amino acid sequences that contain an amino acid (e.g., Met) encoded by an initiation codon at its N-terminus. Therefore, sequences that further comprise an amino acid (e.g., Met) encoded by an initiation codon at the N-terminus of the aforementioned amino acid sequence are also within the scope of protection of the present application.

In certain embodiments, the nucleic acid molecule is a DNA molecule or an RNA molecule.

In certain embodiments, the nucleic acid molecule is an RNA molecule.

In certain embodiments, the nucleic acid molecule comprises a coding sequence of the 3C protease.

In certain embodiments, the nucleic acid molecule further comprises one or more selected from the group consisting of 5'UTR, Kozak sequence, initiation codon, termination codon, 3'UTR, and poly-A tail.

In certain embodiments, the nucleic acid molecule comprises, from the 5' end to the 3' end in the following order: a 5'UTR, a Kozak sequence, an initiation codon, a coding sequence of the 3C protease, a termination codon, a 3'UTR, and a poly-A tail.

According to the codon degeneracy in the art, in certain embodiments, the coding sequence of the 3C protease can be replaced according to the codon degeneracy. In certain embodiments, the coding sequence of the 3C protease is codon-optimized according to the codon bias of a host cell (e.g., a human cell) or not optimized.

In certain embodiments, the coding sequence of the 3C protease has a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 3. In certain embodiments, the coding sequence of the 3C protease is as set forth in SEQ ID NO: 3.

In certain embodiments, the nucleic acid molecule carries a 5' cap modification (e.g., Cap 0, Cap 1, Cap 2) at its 5' end. In certain embodiments, the nucleic acid molecule carries a 5' Cap 1 modification at its 5' end.

In certain embodiments, the nucleic acid molecule comprises one or more N1-methyl pseudouridine (m1$\psi$) modifications. In certain embodiments, some or all the uracil nucleotides in the nucleic acid molecule are replaced with N1-methyl pseudouracil nucleotides.

In certain embodiments, the coding sequence of the 3C protease carries one or more termination codons at its 3' end.

In certain embodiments, the 5'UTR is selected from the group consisting of 5'UTR derived from human $\alpha$-globin mRNA.

In certain embodiments, the 3'UTR is selected from the group consisting of 3'UTR based on mtRNR1 (mitochondrial 12S ribosomal RNA) and AES (Amino-terminal enhancer of split).

In certain embodiments, the 5'UTR has a nucleotide sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 4. In certain embodiments, the 5'UTR has a nucleotide sequence as set forth in SEQ ID NO: 4.

In certain embodiments, the 3'UTR has a nucleotide sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 6. In certain embodiments, the 3'UTR has a nucleotide sequence as set forth in SEQ ID NO: 6.

In certain embodiments, the nucleic acid molecule has a nucleotide sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 2. In certain embodiments, the nucleic acid molecule has a nucleotide sequence as set forth in SEQ ID NO: 2.

In certain embodiments, the Kozak sequence is as set forth in SEQ ID NO: 5.

In certain embodiments, the poly-A tail comprises one or more polyadenylic acid sequences, each of which is independently composed of 20 to 120 consecutive adenylic acids. In certain embodiments, the poly-A tail comprises multiple polyadenylic acid sequences, and the adjacent polyadenylic acid sequences are connected by a spacer sequence comprising non-A.

In certain embodiments, the medicament is for use in a subject to:

(1) inhibit tumor cell proliferation;
(2) inhibit tumor cell migration and/or invasion;
(3) promote tumor cell apoptosis;
(4) downregulate the upstream p38 protein, downstream PSF protein and/or heterogeneous nuclear ribonucleoprotein (hnRNP) A1 in the MNK1/2 signaling pathway in tumor cell;
(5) upregulate the apoptosis execution protein caspase-3 in tumor cell; and/or
(6) inhibit the viability of tumor cell.

In certain embodiments, the tumor is selected from the group consisting of solid tumor and hematological tumor (e.g., leukemia, lymphoma, myeloma).

In certain embodiments, the tumor is selected from the group consisting of glioma (e.g., glioblastoma), liver cancer, kidney cancer, melanoma, prostate cancer, colon cancer, cervical cancer, gastric cancer, lung cancer, colorectal cancer, bladder cancer, breast cancer, uterine/cervical cancer, ovarian cancer, gastrointestinal cancer, pancreatic cancer, skin cancer, leukemia, and sarcoma (e.g., osteosarcoma).

In certain embodiments, the 3C protease or the nucleic acid molecule encoding the 3C protease is administered in combination with an additional pharmaceutically active agent, e.g., administered, simultaneously, separately or sequentially.

In certain embodiments, the additional pharmaceutically active agent is a drug with anti-tumor activity.

In certain embodiments, the additional pharmaceutically active agent is selected from the group consisting of: EGFR inhibitor, HER2 inhibitor, HER3 inhibitor, HER4 inhibitor, IGFR-1 inhibitor, mTOR inhibitor, PI3 kinase inhibitor, c-MET or VEGF inhibitor, antibody drug, biological drug, chemotherapeutic drug and any combination thereof.

In a second aspect, the present application provides an isolated nucleic acid molecule comprising a nucleotide sequence encoding a 3C protease, wherein the 3C protease is as defined in the first aspect.

In certain embodiments, the isolated nucleic acid molecule is the nucleic acid molecule as defined in the first aspect.

In certain embodiments, the isolated nucleic acid molecule encodes the 3C protease as defined in the first aspect.

In certain embodiments, the isolated nucleic acid molecule is a DNA molecule or an RNA molecule.

In certain embodiments, the isolated nucleic acid molecule is an RNA molecule.

In certain embodiments, the isolated nucleic acid molecule comprises a coding sequence of the 3C protease.

In certain embodiments, the isolated nucleic acid molecule further comprises one or more selected from the group consisting of 5'UTR, Kozak sequence, initiation codon, termination codon, 3'UTR, and poly-A tail.

In certain embodiments, the isolated nucleic acid molecule comprises, from the 5' end to the 3' end in the following order: a 5'UTR, a Kozak sequence, an initiation codon, a coding sequence of the 3C protease, a termination codon, a 3'UTR, and a poly-A tail.

In certain embodiments, the coding sequence of the 3C protease is codon-optimized according to the codon bias of a host cell (e.g., a human cell) or not optimized.

In certain embodiments, the coding sequence of the 3C protease has a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 3. In certain embodiments, the coding sequence of the 3C protease is as set forth in SEQ ID NO: 3.

In certain embodiments, the isolated nucleic acid molecule carries a 5' cap modification (e.g., Cap 0, Cap 1, Cap 2) at its 5' end. In certain embodiments, the isolated nucleic acid molecule carries a 5' Cap 1 modification at its 5' end.

In certain embodiments, the isolated nucleic acid molecule comprises one or more N1-methyl pseudouridine (m1ψ) modifications. In certain embodiments, some or all the uracil nucleotides in the isolated nucleic acid molecule are replaced with N1-methylpseudouracil nucleotides.

In certain embodiments, the coding sequence of the 3C protease carries one or more termination codons at its 3' end.

In certain embodiments, the 5'UTR is selected from the group consisting of 5'UTR derived from human α-globin mRNA.

In certain embodiments, the 3'UTR is selected from the group consisting of 3'UTR based on mtRNR1 (mitochondrial 12S ribosomal RNA) and AES (Amino-terminal enhancer of split).

In certain embodiments, the 5'UTR has a nucleotide sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 4. In certain embodiments, the 5'UTR has a nucleotide sequence as set forth in SEQ ID NO: 4.

In certain embodiments, the 3'UTR has a nucleotide sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 6. In certain embodiments, the 3'UTR has a nucleotide sequence as set forth in SEQ ID NO: 6.

In certain embodiments, the isolated nucleic acid molecule has a nucleotide sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 2. In certain embodiments, the isolated nucleic acid molecule has a nucleotide sequence as set forth in SEQ ID NO: 2.

In certain embodiments, the Kozak sequence is as set forth in SEQ ID NO: 5.

In certain embodiments, the poly-A tail comprises one or more polyadenylic acid sequences, each of which is independently composed of 20 to 120 consecutive adenylic acids. In certain embodiments, the poly-A tail comprises multiple polyadenylic acid sequences, and the adjacent polyadenylic acid sequences are connected by a spacer sequence comprising non-A.

In a third aspect, the present application provides a vector comprising the isolated nucleic acid molecule of the second aspect.

In certain embodiments, the vector of the present application is, for example, a plasmid, a cosmid, a bacteriophage, a lentivirus, or the like.

In certain embodiments, the vector is a cloning vector or an expression vector.

In certain embodiments, the vector is capable of expressing the 3C protease as described in the first aspect in a subject (e.g., a mammal, such as a human).

In certain embodiments, the vector is a DNA vector or an RNA vector.

In a fourth aspect, the present application provides a delivery composition comprising a delivery vehicle, and one or more selected from the group consisting of: a 3C protease, the isolated nucleic acid molecule of the second aspect, and a vector of the third aspect,

wherein, the 3C protease is as defined in the first aspect.

In certain embodiments, the delivery vehicle is a particle.

In certain embodiments, the delivery vehicle is selected from the group consisting of lipid particle, sugar particle, metal particle, protein particle, liposome, exosome, microbubble, gene gun and viral vector (e.g., replication-defective retrovirus, lentivirus, adenovirus or adeno-associated virus).

In certain embodiments, the delivery vehicle is a lipid nanoparticle.

In certain embodiments, the lipid nanoparticle comprises an ionizable cationic lipid SM-102, a helper lipid DSPC, a cholesterol and a PEGylated lipid PEG2000-DMG.

In certain embodiments, the molar concentration ratio of the ionizable cationic lipid SM-102 to the helper lipid DSPC in the lipid nanoparticle is in the range of 2:1 to 10:1 (e.g., 2:1 to 5:1, 2:1 to 8:1, 3:1 to 5:1, 3:1 to 8:1, 3:1 to 10:1, 5:1 to 8:1, 5:1 to 10:1). In certain embodiments, the molar concentration ratio of the ionizable cationic lipid SM-102 to the helper lipid DSPC in the lipid nanoparticle is 5:1.

In certain embodiments, the molar concentration ratio of the ionizable cationic lipid SM-102 to the cholesterol in the lipid nanoparticle is in the range of 1:1 to 2:1 (e.g., 1:1 to 1.3:1, 1:1 to 1.5:1, 1.3:1 to 1.5:1, 1.3:1 to 2:1). In certain embodiments, the molar concentration ratio of the ionizable cationic lipid SM-102 to the helper lipid DSPC in the lipid nanoparticle is 50:38.5.

In certain embodiments, the molar concentration ratio of the ionizable cationic lipid SM-102 to the PEGylated lipid PEG2000-DMG in the lipid nanoparticle is in the range of 20:1 to 50:1 (e.g., 20:1 to 30:1, 20:1 to 40:1, 30:1 to 40:1, 30:1 to 50:1). In certain embodiments, the molar concentration ratio of the ionizable cationic lipid SM-102 to the helper lipid DSPC in the lipid nanoparticle is 50:1.5.

In certain embodiments, the molar concentration ratio of the ionizable cationic lipid SM-102, the helper lipid DSPC, the cholesterol, and the PEGylated lipid PEG2000-DMG in the lipid nanoparticle is 50:10:38.5:1.5.

In a fifth aspect, the present application provides a pharmaceutical composition comprising a 3C protease, the isolated nucleic acid molecule of the second aspect, the vector of the third aspect and/or the delivery composition of the fourth aspect, and a pharmaceutically acceptable carrier and/or excipient,

wherein, the 3C protease is as defined in the first aspect.

In certain embodiments, the pharmaceutical composition further comprises an additional pharmaceutically active agent.

In certain embodiments, the additional pharmaceutically active agent is a drug with anti-tumor activity.

In certain embodiments, the additional pharmaceutically active agent is selected from the group consisting of:

EGFR inhibitor, HER2 inhibitor, HER3 inhibitor, HER4 inhibitor, IGFR-1 inhibitor, mTOR inhibitor, PI3 kinase inhibitor, c-MET or VEGF inhibitor, antibody drug, biological drug (e.g., biomacromolecule drug), chemotherapeutic drug, cellular therapeutic agent and any combination thereof.

In certain embodiments, the additional pharmaceutically active agent is provided as a separate component or as a mixed component with the 3C protease, the isolated nucleic acid molecule, the vector and/or the delivery composition.

In a sixth aspect, the present application provides a method for inhibiting a tumor cell, comprising: contacting a 3C protease, the isolated nucleic acid molecule of the second aspect, the vector of the third aspect, the delivery composition of the fourth aspect, or the pharmaceutical composition of the fifth aspect with the tumor cell, or delivering a 3C protease, the isolated nucleic acid molecule of the second aspect, the vector of the third aspect, the delivery composition of the fourth aspect, or the pharmaceutical composition of the fifth aspect to the tumor cell;

wherein, the 3C protease is as defined in the first aspect.

In certain embodiments, the method is for:

(1) inhibiting proliferation of the tumor cell;
(2) inhibiting migration and/or invasion of the tumor cell;
(3) promoting apoptosis of the tumor cell;
(4) downregulating the upstream p38 protein, downstream PSF protein and/or heterogeneous nuclear ribonucleoprotein (hnRNP) A1 in the MNK1/2 signaling pathway in the tumor cell;
(5) upregulating the apoptosis execution protein caspase-3 in the tumor cell; and/or
(6) inhibiting the activity of the tumor cell.

In certain embodiments, the tumor is selected from the group consisting of solid tumor and hematological tumor (e.g., leukemia, lymphoma, myeloma).

In certain embodiments, the tumor is selected from the group consisting of glioma (e.g., glioblastoma), liver cancer, kidney cancer, melanoma, prostate cancer, colon cancer, cervical cancer, gastric cancer, lung cancer, colorectal cancer, bladder cancer, breast cancer, uterine/cervical cancer, ovarian cancer, gastrointestinal cancer, pancreatic cancer, skin cancer, leukemia, and sarcoma (e.g., osteosarcoma).

In certain embodiments, the method is an *in vitro* method or an *in vivo* method. In certain embodiments, the method is an *in vitro* method.

In a seventh aspect, the present application provides a method for the prevention and/or treatment of a tumor in a subject, comprising administering to the subject in need thereof an effective amount of the 3C protease, the isolated nucleic acid molecule of the second aspect, the vector of the third aspect, the delivery composition of the fourth aspect, or the pharmaceutical composition of the fifth aspect.

In certain embodiments, the method is for use in the subject to:

(1) inhibit tumor cell proliferation;
(2) inhibit tumor cell migration and/or invasion;
(3) promote tumor cell apoptosis;
(4) downregulate the upstream p38 protein, downstream PSF protein and/or heterogeneous nuclear ribonucleoprotein (hnRNP) A1 in the MNK1/2 signaling pathway in tumor cell;
(5) upregulate the apoptosis execution protein caspase-3 in tumor cell; and/or
(6) inhibit the viability of tumor cell.

In certain embodiments, the tumor is selected from the group consisting of solid tumor and hematological tumor (e.g., leukemia, lymphoma, myeloma).

In certain embodiments, the tumor is selected from the group consisting of glioma (e.g., glioblastoma), liver cancer, kidney cancer, melanoma, prostate cancer, colon cancer, cervical cancer, gastric cancer, lung cancer, colorectal cancer, bladder cancer, breast cancer, uterine/cervical cancer, ovarian cancer, gastrointestinal cancer, pancreatic cancer, skin cancer, leukemia, and sarcoma (e.g., osteosarcoma).

In certain embodiments, the method further comprises administering to the subject a second therapy, wherein the second therapy is selected from the group consisting of surgery, chemotherapy, radiotherapy, immunotherapy, gene therapy, DNA therapy, RNA therapy, nanotherapy, virotherapy, adjuvant therapy, and any combination thereof; optionally, the second therapy may be administered simultaneously, separately, or sequentially.

In an eighth aspect, the present application provides a use of the isolated nucleic acid molecule of the second aspect, the vector of the third aspect, the delivery composition of the fourth aspect, or the pharmaceutical composition of the fifth aspect in the preparation of a medicament for anti-tumor, or a use of a 3C protease, the isolated nucleic acid molecule of the second aspect, the vector of the third aspect, the delivery composition of the fourth aspect, or the pharmaceutical composition of the fifth aspect in the inhibition of a tumor cell, wherein the 3C protease is as defined in the first aspect.

In certain embodiments, the tumor is selected from the group consisting of solid tumor and hematological tumor (e.g., leukemia, lymphoma, myeloma).

In certain embodiments, the tumor is selected from the group consisting of glioma (e.g., glioblastoma), liver cancer, kidney cancer, melanoma, prostate cancer, colon cancer, cervical cancer, gastric cancer, lung cancer, colorectal cancer, bladder cancer, breast cancer, uterine/cervical cancer, ovarian cancer, gastrointestinal cancer, pancreatic cancer, skin cancer, leukemia, and sarcoma (e.g., osteosarcoma).

The pharmaceutical composition of the present application can be formulated into any dosage form known in the medical field, for example, tablet, pill, suspension, emulsion, solution, gel, capsule, powder, granule, elixir, lozenge, suppository, injection (including solution for injection, sterile powder for injection and concentrated solution for injection), inhalant, spray, and the like. The preferred dosage form depends on the intended mode of administration and therapeutic use. The pharmaceutical composition of the present application should be sterile and stable under the conditions of manufacture and storage. A preferred dosage form is an injection. Such an injection may be a sterile injectable solution. For example, a sterile injectable solution can be prepared by the following method: incorporating a required dose of the pharmaceutical composition of the present application into an appropriate solvent, and optionally, simultaneously incorporating other desired ingredients (including but not limited to, pH regulator, surfactant, adjuvant, ionic strength enhancer, isotonic agent, preservative, diluent, or any combination thereof), followed by filtration and sterilization. In addition, the sterile injectable solution can be prepared as a sterile lyophilized powder (e.g., by vacuum drying or freeze-drying) for easy storage and use. Such a sterile lyophilized powder can be dispersed in a suitable carrier, such as sterile pyrogen-free water, prior to use.

In addition, the 3C protease, the nucleic acid molecule encoding the 3C protease, the vector or the delivery composition of the present application can be present in the pharmaceutical composition in a unit dose form for easy administration.

The pharmaceutical composition of the present application can be administered by any suitable method known in the art, including but not limited to, oral, buccal, sublingual, ocular, topical, parenteral, rectal, intrathecal, intracisternal, inguinal, intravesical, topical (e.g., powder, ointment or drop), or nasal route. However, for many therapeutic uses, the preferred route/mode of administration is parenteral administration (e.g., intravenous injection, subcutaneous injection, intraperitoneal injection, intramuscular injection, intratumoral injection). Those skilled in the art will understand that the route and/or mode of administration will vary depending on the intended purpose. In certain preferred embodiments, the pharmaceutical composition of the present application is administered by intravenous infusion or injection.

The pharmaceutical composition of the present application may comprise a "therapeutically effective amount" or a "prophylactically effective amount" of the 3C protease, the nucleic acid molecule encoding the 3C protease, the vector or the delivery composition of the present application. The term "Prophylactically effective amount" refers to an amount sufficient to prevent, inhibit, or delay the occurrence of a disease. The term "Therapeutically effective amount" refers to an amount sufficient to cure or at least partially inhibit a disease and complications thereof in a patient who already has the disease. The therapeutically effective amount of the 3C protease, the nucleic acid molecule encoding the 3C protease, the vector or the delivery composition of the present application may vary depending on the following factors: the severity of the disease to be treated, the general state of the patient's own immune system, the patient's general condition such as age, weight and gender, the route of administration of drug, and other therapy administered concurrently, etc.

In the present application, the dosage regimen can be adjusted to achieve the optimally desired response (e.g., a therapeutic or prophylactic response). For example, a single administration can be given, multiple administrations may be given over a period of time, or the dose can be proportionally reduced or increased depending on the urgency of the therapeutic situation.

In the present application, the subject can be a mammal, such as a human. In certain embodiments, the subject (e.g., human) suffers from a tumor, or is at risk of suffering from the aforementioned disease.

Definition of terms

In the present application, unless otherwise specified, the scientific and technical terms used herein have the meanings commonly understood by those skilled in the art. In addition, the virology, biochemistry, and immunological laboratory operation steps used herein are all conventional steps widely used in the corresponding fields. Meanwhile, in order to better understand the present application, the definitions and explanations of the relevant terms are provided below.

When the terms "for example," "e.g.," "such as," "comprising," "including" or their variants are used herein, these terms shall not be regarded as restrictive terms, but shall be interpreted as meaning "but not limited to" or "not limited to."

Unless otherwise indicated herein or clearly contradicted by context, the terms "a" and "an" and "the" and similar referents in the context of describing the present application (especially in the context of the following claims) shall be construed to cover both the singular and the plural.

As used herein, the term "and/or" shall be construed as a specific disclosure of each of two or more specified features or elements, as well as any combination of two or more features or elements. Thus, the term "and/or" used in phrases such as "A and/or B" is intended to include "A and B", "A or B", "A" (alone), and "B" (alone).

As used herein, the term "picornavirus" refers to a virus belonging to the family *Picornaviridae,* which is a family of non-enveloped, icosahedral capsid RNA viruses that includes the genera *Enterovirus, Aphthovirus,* and the like.

As used herein, the term "enterovirus" refers to a virus belonging to the genus *Enterovirus,* including poliovirus, coxsackievirus, rhinovirus, enterocytopathic human orphan virus (ECHO, abbreviated as echovirus) and novel enterovirus. Among them, for the serotyping of enteroviruses, reference may be made to Hyypiä T, Hovi T, Knowles NJ, Stanway G. Classification of enteroviruses based on molecular and biological properties. J Gen Virol. 1997;78 (Pt 1):1-11., the entire contents of which are incorporated herein by reference.

As used herein, the term "aphthovirus" refers to a virus belonging to the genus *Aphthovirus,* including foot-and-mouth disease virus, bovine rhinitis virus A, and bovine rhinitis virus B, for example, reference may be made to Schoch, Conrad L et al. "NCBI Taxonomy: a comprehensive update on curation, resources and tools." Database: the journal of biological databases and curation vol. 2020 (2020): baaa062., the entire contents of which are incorporated herein by reference.

As used herein, the term "p38 protein" refers to p38 mitogen-activated protein kinase, which has the meaning generally understood by those skilled in the art. In certain embodiments, the Uniprot ID of the p38 protein described herein is Q15759. In certain embodiments, the p38 protein described herein has: (i) an amino acid sequence corresponding to the aforementioned Uniprot ID, or (ii) an amino acid sequence having a sequence identity of at least 90%, at least 91%, at least 92%, at least 93%, at least 94%, at least 95%, at least 96%, at least 97%, at least 98%, or at least 99% as compared with (i).

As used herein, the term protein "PSF" refers to a PTB (polypyrimidine tract-binding protein)-associated splicing factor, which has the meaning generally understood by those skilled in the art. In certain embodiments, the Uniprot ID of the PSF protein described herein is P23246. In certain embodiments, the PSF protein described herein has: (i) an amino acid sequence corresponding to the aforementioned Uniprot ID, or (ii) an amino acid sequence having a sequence identity of at least 90%, at least 91%, at least 92%, at least 93%, at least 94%, at least 95%, at least 96%, at least 97%, at least 98%, or at least 99% as compared with (i).

As used herein, the term "caspase-3" refers to cysteine-aspartic acid protease 3, which has the meaning generally understood by those skilled in the art. In certain embodiments, the Uniprot ID of the caspase-3 described herein is P42574. In certain embodiments, the caspase-3 described herein has: (i) an amino acid sequence corresponding to the aforementioned Uniprot ID, or (ii) an amino acid sequence having a sequence identity of at least 90%, at least 91%, at least 92%, at least 93%, at least 94%, at least 95%, at least 96%, at least 97%, at least 98%, or at least 99% as compared with (i).

As used herein, the term "identity" refers to the degree of sequence match between two polypeptides or between two nucleic acids. When two sequences for comparison have the same monomer sub-unit of base or amino acid at a certain site (e.g., each of two DNA molecules has an adenine at a certain site, or each of two polypeptides has a lysine at a certain site), the two molecules are identical at the site. The "percent identity" between two sequences is a function of the number of identical sites shared by the two sequences over the total number of sites for comparison $\times$ 100. For example, if 6 of 10 sites of two sequences are matched, these two sequences have an identity of 60%. For example, DNA sequences: CTGACT and CAGGTT share an identity of 50% (3 of 6 sites are matched). Generally, the comparison is performed when the two sequences are aligned to yield maximum identity. Such alignment can be achieved using, for example, the method of Needleman et al. (J. Mol. Biol. 48:443-453, 1970), which can be conveniently carried out by computer programs such as the Align program (DNAstar, Inc.). The percent identity between two amino acid sequences can also be determined using the algorithm of E. Meyers and W. Miller (Comput. Appl. Biosci., 4:11-17 (1988)) which has been incorporated into the ALIGN program (version 2.0), using a PAM120 weight residue table, and with a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and with a gap weight of 16, 14, 12, 10, 8, 6, or 4 and a length weight of 1, 2, 3, 4, 5, or 6.

As used herein, the term "3'UTR based on mtRNR1 (mitochondrial 12S ribosomal RNA) and AES (amino-terminal enhancer of split)" has the meaning commonly understood by those skilled in the art, and generally refers to a 3'UTR comprising a mitochondrial 12S rRNA-related sequence and an AES (amino-terminal enhancer of split) mRNA sequence. For example, an exemplary 3'UTR can be found in Orlandini von Niessen AG, et al. Improving mRNA-Based Therapeutic Gene Delivery by Expression-Augmenting 3' UTRs Identified by Cellular Library Screening. Mol Ther. 2019 Apr 10;27(4):824-836. doi: 10.1016/j.ymthe.2018.12.011., the entire contents which are incorporated herein by reference.

The writing of the twenty conventional amino acids involved herein follows conventional usage. See, for example, Immunology-A Synthesis (2nd Edition, E. S. Golub and D. R. Gren, Eds., Sinauer Associates, Sunderland, Mass. (1991)), which is incorporated herein by reference. In the present application, the terms "polypeptide" and "protein" have the same meaning, and are used interchangeably. Furthermore, in the present application, amino acids are generally represented by single-letters and three-letter abbreviations well known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995), and includes, but is not limited to: pH regulator, surfactant, adjuvant, ionic strength enhancer, diluent, agent for maintaining osmotic pressure, agent for delaying absorption, preservative. For example, the pH regulator includes but is not limited to phosphate buffer. The surfactant includes but is not limited to cationic, anionic or non-ionic surfactant, such as Tween-80. The ionic strength enhancer includes but is not limited to sodium chloride. The preservative includes but is not limited to various antibacterial agents and antifungal agents, such as paraben, chlorobutanol, phenol, sorbic acid, and the like. The agent for maintains osmotic pressure includes, but is not limited to, sugar, NaCl and the like. The agent for delaying absorption includes, but is not limited to, monostearate and gelatin. The diluent includes, but is not limited to, water, aqueous buffer (e.g., buffered saline), alcohol and polyol (e.g., glycerol), and the like. The term "stabilizer" has the meaning commonly understood by those skilled in the art, which is capable of stabilizing the desired activity of the active ingredient in a drug, includes but not limited to sodium glutamate, gelatin, SPGA, sugar (e.g., sorbitol, mannitol, starch, sucrose, lactose, dextran, or glucose), amino acid (e.g., glutamic acid, glycine), protein (e.g., dried whey, albumin or casein) or degradation product thereof (e.g., lactalbumin hydrolysate), and the like.

As used herein, the term "prevention" refers to a method implemented to prevent or delay the occurrence of a disease or condition or symptom (e.g., a tumor) in a subject. As used herein, the term "treatment" refers to a method implemented to obtain a beneficial or desired clinical result. For the purposes of the present application, beneficial or desired clinical results include, but are not limited to, alleviation of symptoms, diminishment of the extent of the disease, stabilization (i.e., no longer worsening) of the state of the disease, delay or slowing of the progression of the disease, improvement or alleviation of the state of the disease, and relief of symptoms (whether partial or complete), whether detectable or undetectable. In addition, "treatment" may also refer to prolongation of survival as compared to the expected survival if not receiving treatment.

The terms "cancer" and "tumor" are used interchangeably and refer to a broad class of diseases characterized by the uncontrolled growth of abnormal cells in the body. Uncontrolled cell division may lead to the formation of malignant tumors or cells that invade adjacent tissues and may metastasize to distant parts of the body through the lymphatic system or bloodstream. Cancers include benign and malignant cancers as well as dormant tumors or micrometastases. Cancers also include hematological malignances.

Beneficial effects of the invention

The method for the prevention and/or treatment of a tumor provided in the present application, which is based on a picornavirus (e.g., *Enterovirus 71, Coxsackievirus A14)* 3C protease or a nucleic acid molecule encoding the 3C protease, exhibit broad-spectrum and significant tumor cell-killing activity, and has great clinical application value.

The embodiments of the present application will be described in detail below with reference to the drawings and examples. However, those skilled in the art will understand that the following drawings and examples are for illustrative purposes only and not intended to limit the scope of the present application. According to the following detailed description of the drawings and preferred embodiments, the various objects and advantages of the present application will become apparent to those skilled in the art.

**Brief Description of the Drawings**

Figure 1 shows the vector sequence and *in vitro* transcription vector of EV-3C-mRNA.

Figure 2 shows that EV-3C-mRNA can inhibit the proliferation of glioblastoma cells.

Figure 3 shows that EV-3C-mRNA inhibits the migration and invasion of glioblastoma cell lines.

Figure 4 shows that EV-3C protein induces the upregulation of Cleaved Caspase-3 and activates the apoptotic signaling pathway.

Figure 5 shows that EV-3C-mRNA can induce the apoptosis of human glioblastoma cells.

Figure 6 shows that EV-3C protein downregulates multiple proteins in the MNK1/2 signaling pathway.

Figure 7 shows that EV-3C-mRNA can inhibit the cell viability of glioblastoma cells.

Figure 8 shows the schematic diagram and physical characterization of lipid nanoparticle (LNP) encapsulation of EV-3C-mRNA.

Figure 9 shows the *in vivo* delivery ability evaluation of lipid nanoparticles.

Figure 10 shows that EV-3C-mRNA-LNP can inhibit the growth of *in situ* brain tumors in mice and prolong the survival time of mice.

Figure 11 shows the *in vivo* safety evaluation of EV-3C-mRNA lipid nanoparticles.

Figure 12 shows the evaluation of EV-3C-mRNA on cell viability of other tumor cell lines.

Figure 13 shows the $IC_{50}$ inhibition rate and therapeutic index of EV-3C-mRNA on different cell lines.

Sequence Information

The description of the sequences involved in the present application is provided in the following table.

Table 1: Sequence information

| SEQ ID NO: | Sequences and descriptions |
|---|---|
| 1 | Amino acid sequence of EV-3C<br><br>GPSLDFALSLLRRNIRQVQTDQGHFTMLGVRDRLAVLPRHSQPGKTIWIEHKLV NVLDAVELVDEQGVNLELTLITLDTNEKFRDITKFIPENISTASDATLVINTEHMP SMFVPVGDVVQYGFLNLSGKPTHRTMMYNFPTKAGQCGGVVTSVGKVIGIHIG GNGRQGFCAGLKRSYFASEQ |
| 2 | Nucleotide sequence of EV-3C-mRNA<br><br>GGGAAUAAACUAGUAUUCUUCUGGUCCCCACAGACUCAGAGAGAACCCGCCA CCAUGGGCCCCAGCCUGGACUUCGCCCUGAGCCUGCUGCGGAGAAAUAUCCGG CAGGUGCAGACCGAUCAAGGCCACUUUACAAUGCUGGGCGUGAGAGACCGGC UCGCCGUGCUGCCUCGCCACUCCCAGCCUGGAAAGACCAUUUGGAUCGAGCAU AAGCUGGUGAACGUGCUGGAUGCCGUGGAACUGGUCGACGAGCAGGGCGUGA ACCUGGAGCUGACACUGAUCACCCUGGACACCAACGAGAAGUUCCGGGACAUC ACCAAAUUCAUCCCCGAAAACAUCAGCACCGCCUCCGAUGCUACACUGGUGAU CAACACCGAGCACAUGCCUAGCAUGUUCGUGCCCGUGGGCGACGUGGUGCAG UACGGCUUCCUGAACCUGAGCGGCAAGCCAACCCACCGGACAAUGAUGUACAA UUUCCCUACAAAGGCCGGACAGUGCGGCGGGGUGGUGACCUCUGUGGGCAAG GUGAUCGGCAUCCACAUCGGCGGCAACGGAAGGCAGGGCUUUUGUGCCGGCC UGAAGAGAAGCUACUUCGCCAGCGAGCA**GUGAUGACUCGAGCUGGUACUGCA UGCACGCAAUGCUAGCUGCCCCUUUCCCGUCCUGGGUACCCCGAGUCUCCCCC GACCUCGGGUCCCAGGUAUGCUCCCACCUCCACCUGCCCCACUCACCACCUCU GCUAGUUCCAGACACCUCCCAAGCACGCAGCAAUGCAGCUCAAAACGCUUAGC CUAGCCACACCCCCACGGGAAACAGCAGUGAUUAACCUUUAGCAAUAAACGA AAGUUUAACUAAGCUAUACUAACCCCAGGGUUGGUCAAUUUCGUGCCAGCCA CACCCUGGAGCUAGC**AAAAAAAAAAAAAAAAAAAAAAAAAAAAAAAGCAUAUG ACUAAAAAAAAAAAAAAAAAAAAAAAAAAAAAAAAAAAAAAAAAAAAAAAA AAAAAAAAAAAAAAAAAAAAAA |

(continued)

| SEQ ID NO: | Sequences and descriptions |
|---|---|
| 3 | Nucleotide sequence encoding of EV-3C<br><br>GGCCCCAGCCUGGACUUCGCCCUGAGCCUGCUGCGGAGAAAUAUCCGGCAG GUGCAGACCGAUCAAGGCCACUUUACAAUGCUGGGCGUGAGAGACCGGCU CGCCGUGCUGCCUCGCCACUCCCAGCCUGGAAAGACCAUUUGGAUCGAGCA UAAGCUGGUGAACGUGCUGGAUGCCGUGGAACUGGUCGACGAGCAGGGCG UGAACCUGGAGCUGACACUGAUCACCUGGACACCAACGAGAAGUUCCGG GACAUCACCAAAUUCAUCCCCGAAAACAUCAGCACCGCCUCCGAUGCUACA CUGGUGAUCAACACCGAGCACAUGCCUAGCAUGUUCGUGCCCGUGGGCGAC GUGGUGCAGUACGGCUUCCUGAACCUGAGCGGCAAGCCAACCCACCGGACA AUGAUGUACAAUUUCCCUACAAAGGCCGGACAGUGCGGCGGGGUGGUGAC CUCUGUGGGCAAGGUGAUCGGCAUCCACAUCGGCGGCAACGGAAGGCAGG GCUUUUGUGCCGGCCUGAAGAGAAGCUACUUCGCCAGCGAGCAG |
| 4 | 5' UTR<br>GGGAAUAAACUAGUAUUCUUUCUGGUUCCCCACAGACUCAGAGAGAACCC |
| 5 | Kozak sequence<br>GCCACC |
| 6 | 3' UTR<br><br>CUCGAGCUGGUACUGCAUGCACGCAAUGCUAGCUGCCCCUUUCCCGUCCUG GGUACCCCGAGUCUCCCCCGACCUCGGGUCCCAGGUAUGCUCCCACCUCCA CCUGCCCCACUCACCACCUCUGCUAGUUCCAGACACCUCCCAAGCACGCAG CAAUGCAGCUCAAAACGCUUAGCCUAGCCACACCCCCACGGGAAACAGCAG UGAUUAACCUUUAGCAAUAAACGAAAGUUUAACUAAGCUAUACUAACCCC AGGGUUGGUCAAUUUCGUGCCAGCCACACCCUGGAGCUAGC |
| 7 | Poly-A tail<br><br>AAAAAAAAAAAAAAAAAAAAAAAAAAAAAAGCAUAUGACUAAAAAAAAA AAAAAAAAAAAAAAAAAAAAAAAAAAAAAAAAAAAAAAAAAAAAAAAAA AAAAAAAAAA |

**Specific Models for Carrying Out the present application**

The present application is now described with reference to the following examples which are intended to illustrate the present application (but not to limit the present application).

Unless otherwise specified, the molecular biology experimental methods and immunoassays used in the present application were generally carried out in accordance with the methods described in Molecular Cloning: A Laboratory Manual, 2nd Edition, Cold Spring Harbor Laboratory Press, 1989, by J. Sambrook et al., and Short Protocols in Molecular Biology, 3rd Edition, John Wiley & Sons, Inc., 1995, by F. M. Ausubel et al.; the use of restriction endonucleases was in accordance with the conditions recommended by the product manufacturer. Those skilled in the art will understand that the examples describe the present application by way of illustration only and are not intended to limit the scope of the claimed subject matter of the present application.

Example 1: *In vitro* transcription and synthesis of EV-3C-mRNA

Experimental materials: SapI restriction endonuclease used in the experiment was purchased from NEB (Cat. No.: R0569S), T7 RNA polymerase was purchased from Thermo (Cat. No.: EP0111), ATP was purchased from Thermo (Cat. No.: R0441), CTP was purchased from Thermo (Cat. No.: 18331017), GTP was purchased from Thermo (Cat. No.: 18332015), UTP was purchased from Thermo (Cat. No.: R1471), Nuclease-free water was purchased from Thermo (Cat. No.: 10977015), Capping buffer was purchased from Thermo (Cat. No.: J62446.AP), S-adenosyl methionine (SAM) was purchased from NEN (Cat. No.: B9003S), Vaccinia mRNA capping enzyme was purchased from NEB (Cat. No.: M2080S), DNase I was purchased from Thermo (Cat. No.: 18047019), Dynabeads 1.5×RNA Binding Buffer was purchased from Yeasen (Cat. No.: A33562), Dynabeads Myone Carboxylic Acid was purchased from Yeasen (Cat. No.: 65011), and Ambion™ RNA storage solution was purchased from Thermo (Cat. No.: AM7000). The gene amplification instrument was purchased from Hangzhou Bio-Gener Technology Co., Ltd. (Model: AUTO-96), the Ultra Microvolume UV-Vis spectrophotometer was purchased from Thermo (Cat. No.: 840-317400), the thermostatic mixer (refrigerated) was purchased from Kylin-Bell Lab Instruments (Cat. No.: BE-3200), the refrigerated centrifuge was purchased from Germany/Eppendorf (Model: 5424R), and the dry bath incubator was purchased from Hangzhou Allsheng Instruments Co., Ltd. (model: K30).

The experimental scheme was as follows:

(1) Generation of DNA template

The template sequence was designed by comprehensively considering codon bias, GC content and thermodynamic stability of RNA secondary structure, and then the DNA sequence of the coding region of EV-3C (whose amino acid sequence was as set forth in SEQ ID NO: 1, which was derived from EV71 or *Coxsackievirus A14*) was cloned into the *in vitro* transcription vector pmRVac to generate a DNA template for *in vitro* transcription. The pmRVac vector contained a T7 promoter sequence, a 5'UTR, a 3'UTR, a 110 nt segmented polyA and a SapI restriction endonuclease site, with the insertion position located between the UTRs. The reaction product was transformed into *Escherichia coli* and plated on LB plates containing kanamycin. The resulting colonies were screened by PCR to confirm positive insertion events. PCR-positive clones were further validated by restriction endonuclease digestion and Sanger sequencing. The correct plasmids were cultured in LB medium and purified using an endotoxin-free plasmid Midprep kit. The concentration and purity of plasmid DNA were determined by a UV-visible spectrophotometer, followed by linearization with SapI restriction endonuclease.

(2) Synthesis of mRNA

Cap1 mRNA was generated by T7 *in vitro* transcription and subsequent enzymatic capping and methylation. To prepare uncapped RNA transcripts, an *in vitro* transcription reaction was set up by mixing linear DNA templates with T7 RNA polymerase, nucleoside triphosphates (NTPs), and a magnesium-containing buffer. The reaction mixture was incubated at 37°C for 2 hours. For the synthesis of N1-methyl pseudouridine (m1$\psi$)-modified transcripts, m1$\psi$TP (N1-methyl-pseudouridine triphosphate) was used to replace UTP in the *in vitro* transcription.

Cap1 mRNA was prepared using the vaccinia capping system. Uncapped transcripts were heat-denatured, and then a capping reaction was performed by adding vaccinia virus capping enzyme, 2'-O methyltransferase, GTP, S-adenosylmethionine (SAM) and capping buffer. The reaction mixture was incubated at 37°C for 1 hour. Subsequently, the template DNA was removed by DNase I treatment. The capped transcripts were further purified using magnetic beads. The reaction mixture was incubated at 37°C for 1 hour. Subsequently, the template DNA was removed by DNase I treatment. The capped transcripts were further purified using magnetic beads. The isolated mRNA was eluted with an acidic buffer and stored at -80°C. The concentration and purity of mRNA were determined by a UV-visible spectrophotometer. The mRNA integrity was determined by denaturing agarose gel electrophoresis. Finally, mRNA sequencing was performed to identify the mRNA molecules. The nucleotide sequence of the finally obtained mRNA molecule was as set forth in SEQ ID NO: 2, wherein the coding sequence of EV-3C was as set forth in SEQ ID NO: 3.

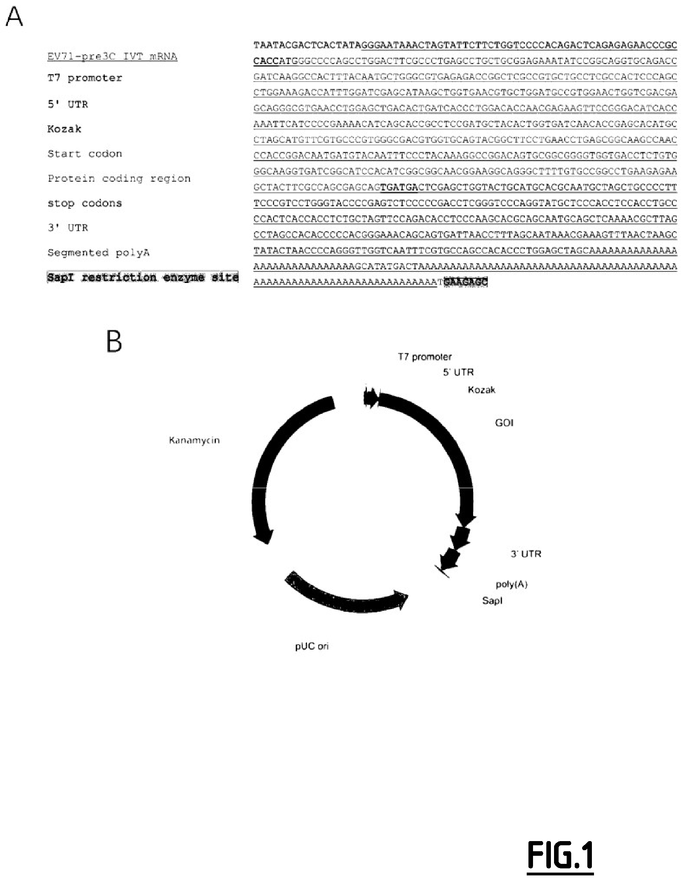

The sequence of the *in vitro* transcription vector was shown in Panel A of Figure 1, and Panel B showed a schematic diagram of the *in vitro* transcription (IVT) vector backbone. The nucleic acid sequence was confirmed by Sanger sequencing, and the results showed that the insert sequence in the plasmid backbone was 100% identical to the reference sequence. The mRNA concentration was quantified using a UV-visible spectrophotometry and determined to be 1553 ng/$\mu$L. The OD260/280 ratio, determined by UV-visible spectrophotometry, was 2.06.

Example 2: Effect of EV-3C-mRNA on cell proliferation detected by the EdU assay

This example was performed based on the EV-3C-mRNA prepared in Example 1.

Experimental materials: The human glioblastoma cells U87 MG used in the experiment were purchased from ATCC (Cat. No.: HTB-14), A172 were purchased from ATCC (Cat. No.: CRL-1620), and human embryonic lung fibroblasts MRC-5 were purchased from ATCC (Cat. No.: CCL-171). The above cells were cultured in a cell incubator at 37°C with 5% $CO_2$, and the complete media used for cell growth were DMEM medium with high glucose (purchased from Gibco, Cat. No.: 11995065) and MEM medium (purchased from Gibco, Cat. No.: 10370021), which were supplemented with 10% fetal bovine serum (purchased from Gibco, Cat. No.: 16000044) and penicillin-streptomycin double antibiotics (purchased from Gibco, Cat. No.: 2321152). BeyoClick™ EdU Cell Proliferation Kit with Alexa Fluor 488 (BeyoClick™ EdU-488) was purchased from Beyotime (Cat. No.: C0071S), and Lipofectamine® 2000 was purchased from Thermo Fisher Scientific (Cat. No.: 11668-019).

The experimental scheme was as follows:

The detection of cell proliferation ability is a basic method for evaluating cell activity, genotoxicity and the efficacy of anti-tumor drug. The most accurate and widely accepted method for detecting cell proliferation is to directly detect the synthesis of DNA in cells. In the present application, BeyoClick™ EdU-488 cell proliferation kit was used according to the literature. EdU represented 5-ethynyl-2'-deoxyuridine. EdU was a thymidine nucleoside analog that could replace T to incorporate into the replicating DNA molecule during the cell proliferation period. The DNA replication activity was detected based on the specific reaction between EdU and Apollo® fluorescent dye. By detecting EdU labeling, the proliferation of cells could be accurately reflected.

Cells were cultured at a density of $5 \times 10^5$ cells per well in a 6-well plate. After the cells were cultured overnight and recovered to a normal state, the medium was replaced, and the transfection of EV-3C-mRNA was performed. 2.5μg, 3μg, and 3.5μg of 3C-mRNA was transfected into U87 MG cells, A172 cells, and MRC5 cells using Lipofectamine® 2000 transfection reagent, respectively. Following incubation for 24 hours, EdU labeling was conducted. The 2× EdU solution was pre-warmed and then added to an equal volume of medium containing the experimental cells to obtain a 1× EdU solution. The treated cells were incubated in an incubator at 37°C for 2 hours, and then the cells were fixed and permeabilized. A reaction solution was prepared for EdU detection, and the Click reaction was performed in a 6-well plate, in which each well contained 430μL of Click Reaction Buffer, 20μL of $CuSO_4$, 1μL of Azide 488, 50μL of Click Additive Solution to form a system with a total volume of 500μL. Incubation was performed at room temperature in the dark for 30 minutes, then the Click reaction solution was removed by pipetting, and the cells were washed 3 times with washing solution for 3 to 5 minutes each time. In order to determine the proportion of cell proliferation, nucleus staining was performed using Hoechst 33342. A 1× Hoechst 33342 solution was prepared by diluting Hoechst 33342 (1000×) in PBS at a ratio of 1:1000. 1 ml of 1× Hoechst 33342 solution was added to each well, followed by incubation at room temperature in the dark for 10 minutes. The 1× Hoechst 33342 solution was then removed by pipetting. The cells were washed 3 times with washing solution for 3 to 5 minutes each time, and then fluorescence detection was performed. Hoechst 33342 emits a blue fluorescence with a maximum excitation wavelength of 346nm and a maximum emission wavelength of 460nm.

Data analysis: Statistical significance was calculated using analysis of variance (ANOVA). Data were presented in the form of mean ± standard error. $p < 0.05$ was considered statistically significant.

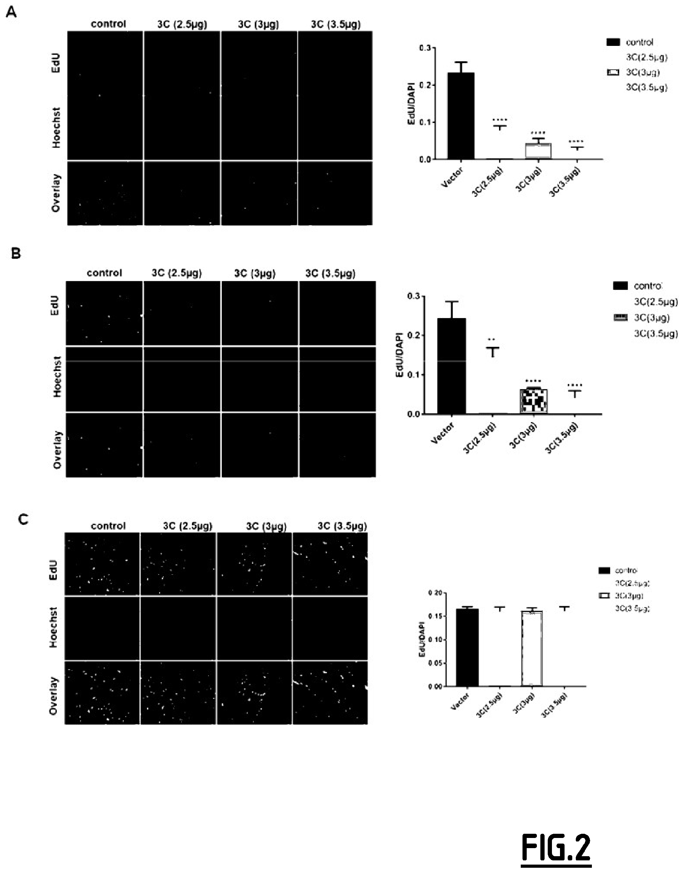

The results of the EdU cell proliferation assays for EV-3C-mRNA in different cell lines were shown in Figure 2 and Table 2. In Figure 2, Panel A showed that the EdU/DAPI ratio for cell proliferation in U87 MG cells decreased significantly with increasing doses of EV-3C-mRNA transfection; Panel B showed that the EdU/DAPI ratio for cell proliferation in A172 cells decreased in a dose-dependent manner with increasing doses of EV-3C-mRNA transfection; and Panel C showed that the EdU/DAPI ratio for cell proliferation in MRC5 cells remained did not change with increasing doses of EV-3C-mRNA transfection.

In summary, EV-3C-mRNA could inhibit the proliferation of various glioblastomas cell lines in a dose-dependent manner.

Table 2. EdU Cell Proliferation Assay Results of EV-3C-mRNA in Different Cell Lines

| EdU/DAPI | Control | 3C (2.5μg) | 3C (3μg) | 3C (3.5μg) |
|---|---|---|---|---|
| U87-MG | 0.204334 | 0.089431 | 0.035088 | 0.02 |
| | 0.225131 | 0.08046 | 0.035714 | 0.034483 |
| | 0.264151 | 0.066667 | 0.058824 | 0.018182 |
| A172 | 0.242424 | 0.138889 | 0.0625 | 0.030303 |
| | 0.2 | 0.125 | 0.058824 | 0.0625 |
| | 0.285714 | 0.171429 | 0.066667 | 0.032258 |
| MRC5 | 0.166976 | 0.16875 | 0.161905 | 0.164 |
| | 0.158009 | 0.150718 | 0.167488 | 0.16849 |
| | 0.169312 | 0.162455 | 0.153242 | 0.150115 |

Example 3: Evaluation of the effects of EV-3C-mRNA on migration and invasion of human glioblastoma cell lines using Transwell assay

This example was performed based on the EV-3C-mRNA prepared in Example 1.

Experimental materials: The Human glioblastoma cells U87 MG used in the experiment was purchased from ATCC (Cat. No.: HTB-14), A172 was purchased from ATCC (Cat. No.: CRL-1620), and human embryonic lung fibroblasts MRC-5 were purchased from ATCC (Cat. No.: CCL-171). The above cells were cultured in a cell incubator at 37°C with 5%

$CO_2$. The complete media used for cell growth were DMEM medium with high glucose (purchased from Gibco, Cat. No.: 11995065) and MEM medium (purchased from Gibco, Cat. No.: 10370021), which were supplemented with 10% fetal bovine serum (purchased from Gibco, Cat. No.: 16000044) and penicillin-streptomycin double antibiotics (purchased from Gibco, Cat. No.: 2321152). Corning® Transwell® (Cat. No.: 07200174); Corning Matrigel® Matrix (Cat. No.: 356234).

The experimental scheme was as follows:

In the cell migration and invasion assay, the Transwell insert was placed in a culture plate, the Transwell chamber was called the upper chamber, and the culture plate was called the lower chamber. The culture media of the upper and lower layers were separated by a polycarbonate membrane. The cells were seeded in the upper chamber. Due to the permeability of the polycarbonate membrane, the cells transfected with EV-3C-mRNA and the control cells were seeded in the upper chambers, and the cells that migrated to the lower membranes were stained and photographed and recorded to study the effects of EV-3C-mRNA on cell migration and cell invasion.

1. U87 MG and A172 cells were transfected with different concentrations of EV-3C-mRNA and incubated overnight for 12 hours. The cells were harvested and resuspended in FBS-free medium.

2. Cells ($1\times10^4$ to $2\times10^4$ cells per well) were seeded in the upper chambers of the Transwell plate (the upper chambers of the Transwell for cell invasion were pre-coated with Matrigel). 0.6ml of DMEM containing 10% fetal bovine serum was added as a chemoattractant to the plate wells (lower chambers) and incubated at 37°C for 16 hours.

3. The cells were fixed with methanol for 15 minutes, and then stained with 1% crystal violet for 20 minutes. The membrane was washed in PBS for a few seconds to remove excess dye.

4. The membrane was dried completely, and the cells were counted under a microscope. Different views were randomly selected, and the average count was taken.

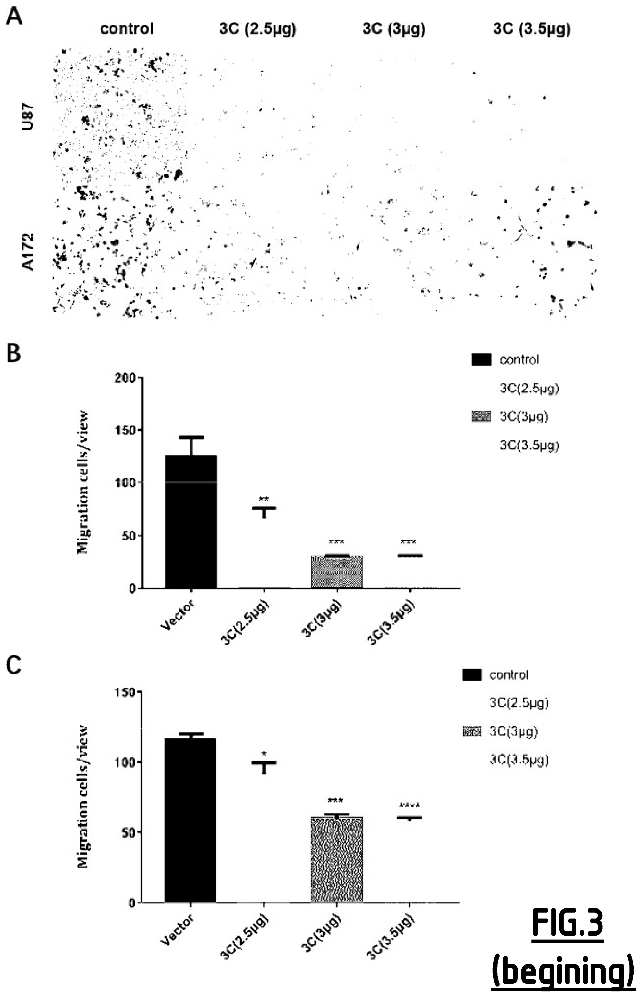

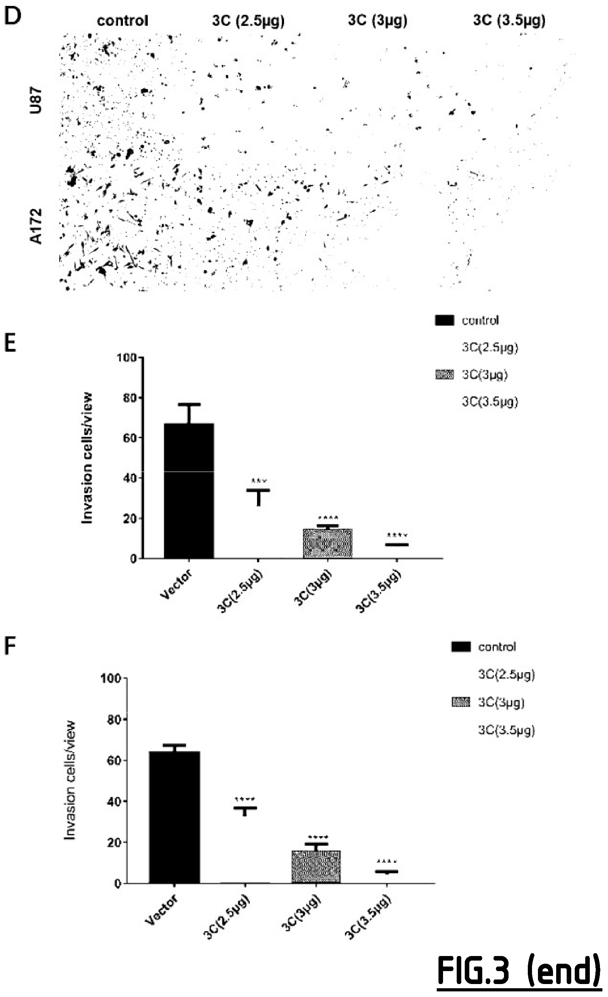

The inhibitory effects of EV-3C-mRNA on the migration and invasion of glioblastoma cell lines were shown in Figure 3 and Table 3. As shown in Panel A of Figure 3, the number of U87 MG and A172 cells that migrated from the upper chamber to the lower chamber gradually decreased with increasing transfection dose. Panels B and C showed the cell counts of U87 MG and A172 cells under microscope fields of view, respectively. Panel D showed that the number of U87 MG and A172 cells that invaded from the upper chamber to the lower chamber gradually decreased with increasing transfection doses. Panels E and F showed the corresponding cell counts of U87 MG and A172 cells under microscope fields of view, respectively.

In summary, EV-3C-mRNA could inhibit the migration and invasion of various glioblastoma cell lines in a dose-dependent manner.

Table 3. Verification of inhibitory activity of EV-3C-mRNA on migration and invasion in different cell lines via Transwell assay

| Migration assay | Control | 3C (2.5μg) | 3C (3μg) | 3C (3.5μg) |
|---|---|---|---|---|
| | 160 | 86 | 32 | 26 |
| U87/number of migrated cells | 100 | 57 | 30 | 30 |
| | 116 | 56 | 28 | 31 |
| | 115 | 100 | 57 | 63 |
| A172/number of migrated cells | 124 | 99 | 66 | 56 |
| | 110 | 76 | 58 | 57 |
| Invasion assay | Control | 3C (2.5μg) | 3C (3μg) | 3C (3.5μg) |
| | 62 | 21 | 12 | 5 |
| U87/number of invasive cells | 78 | 22 | 15 | 6 |
| | 59 | 35 | 16 | 7 |
| | 60 | 32 | 16 | 3 |
| A172/number of invasive cells | 64 | 37 | 19 | 6 |
| | 67 | 30 | 12 | 4 |

Example 4: Detection of activity of EV-3C-mRNA in inducing apoptosis of human glioblastoma cells by immunofluorescence assay

Experimental materials: The human glioblastoma cells U87 MG used in the experiment were purchased from ATCC (Cat. No.: HTB-14), and A172 were purchased from ATCC (Cat. No.: CRL-1620). The above cells were cultured in a cell incubator at 37°C with 5% $CO_2$. The complete media used for cell growth were DMEM medium with high glucose (purchased from Gibco, Cat. No.: 11995065) and MEM medium (purchased from Gibco, Cat. No.: 10370021), which were supplemented with 10% fetal bovine serum (purchased from Gibco, Cat. No.: 16000044) and penicillin-streptomycin double antibiotics (purchased from Gibco, Cat. No.: 2321152). Enterovirus 71 3C Antibody (B3) was purchased from Gene Tex (Cat. No. GTX630191); Cleaved Caspase-3 (Asp175) Antibody was purchased from CST (Cat. No.: #9661); Anti-Rabbit IgG (H+L) Alexa Flour was purchased from Invitrogen (Cat. No.: A16098); Anti-Mouse IgG (H+L) Alexa Flour was purchased from Invitrogen (Cat. No.: A16013).

The experimental scheme was as follows:
On the first day, U87 MG and A172 cells were seeded in black-wall clear-bottom 96-well plates at $1.0 \times 10^4$ cells/-well/100μL, and incubated in a cell incubator at 37°C with 5% $CO_2$ overnight for adherence. On the second day, the pre-seeded black-wall clear-bottom 96-well plates were taken out of the cell incubator, and the medium was replaced with 200μL. Cells in the experimental group were transfected with EV-3C-mRNA (n=6; 100 ng/well) and incubated in the cell incubator overnight. The samples were fixed with 100 μL/well of 4% formaldehyde (final concentration) for 0.5h. The solution was removed, and the cells were washed 3 times with PBS at 200μL/well. The solution was removed, and 200 μL/well of PBS containing 1 μM (final concentration) Hoechst33342 (nuclear dye, blue fluorescence) was added, and incubated at 37°C for 20 to 30min. The solution was removed, and 100 μL/well of 1× permeabilization solution (PBS containing 0.1% Triton X-100) was added, and incubated at room temperature in the dark for 0.5 h. The solution was removed; the cells were washed 3 times with PBS at 200μL/well. The solution was removed, 100μL/well of 1× blocking solution (PBS solution containing 5% BSA) was added, and incubated at 37°C for 0.5h. EV-3C antibody and Cleaved Caspase-3 antibody were prepared by diluting the antibodies 400 times in 1× blocking solution, and mixing thoroughly. The solution was removed, 40μL/well of the above-diluted primary antibody working solution was added with paying attention to avoid bubbles, and incubated at room temperature in the dark for 2h, or incubated at 37°C in the dark for 1h. The primary antibody was recovered, and the cells were washed 3 times with 100μL/well 1× blocking solution. Sufficient secondary antibodies were prepared by diluting corresponding Anti-Rabbit IgG (H+L) Alexa Flour series secondary antibodies and Anti-Mouse IgG (H+L) Alexa Flour series secondary antibodies 500-fold in 1× blocking solution and mixing thoroughly. Yhe solution was removed, 50μL/well of the secondary antibody working solution was added with paying attention to ensure that the fluorescence color of the selected secondary antibody did not overlap with other colors in this experiment, and paying attention to avoid bubbles, and incubated at room temperature in the dark for 1h, or incubated at 37°C in the dark for 0.5h. The solution was removed, the cells were washed three times with 200μL/well of PBS, then 200μL/well of PBS was added; and the detection was performed on a machine.

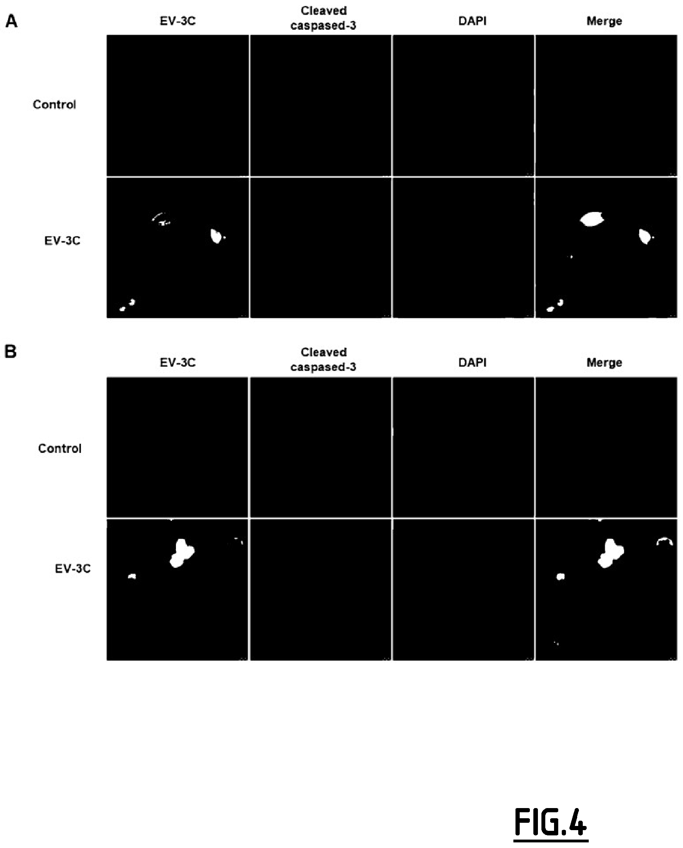

The results were shown in Figure 4. The results showed that transfection with EV-3C-mRNA induced the upregulation of Cleaved Caspase-3 and activated the apoptotic signaling pathway. Panel A showed the result of immunofluorescence staining in U87 MG cells transfected with EV-3C-mRNA for 24 hours, EV-3C protein was labeled with green fluorescence, and Cleaved Caspase-3 is labeled with red fluorescence. Furthermore, the signals for 3C protein and Cleaved Caspase-3 protein overlap in the merged image. Panel B showed the result of fluorescence staining in A172 cells. From the significant contrast between the EV-3C-mRNA transfection group and the PBS control group in both cell lines, it could be seen that 3C protein could induce cellular apoptosis and activate Cleaved Caspase-3 protein.

In summary, EV-3C-mRNA promoted apoptosis of glioblastoma by inducing upregulation of Cleaved Caspase-3.

Example 5: Detection of activity of EV-3C-mRNA in promotion of apoptosis of human glioblastoma cells by Annexin V-FITC apoptosis assay

This example was performed based on the EV-3C-mRNA prepared in Example 1.

Experimental materials: The human glioblastoma cells U87 MG used in the experiment were purchased from ATCC (Cat. No.: HTB-14), A172 were purchased from ATCC (Cat. No.: CRL-1620), and U118MG cells were purchased from ATCC (Cat. No.: HTB-15). The above cells were cultured in a cell incubator at 37°C with 5% $CO_2$. The complete media used for cell growth were DMEM medium with high glucose (purchased from Gibco, Cat. No.: 11995065) and MEM medium (purchased from Gibco, Cat. No.: 10370021), which were supplemented with 10% fetal bovine serum (purchased from Gibco, Cat. No.: 16000044) and penicillin-streptomycin double antibiotics (purchased from Gibco, Cat. No.: 2321152). Annexin V-FITC apoptosis detection kit was purchased from Beyotime (Cat. No.: C1062M).

The experimental scheme was as follows:
Phosphatidylserine is mainly distributed on the inner side of the cell membrane, that is, the side adjacent to the cytoplasm. In the early stage of apoptosis, different types of cells externalize phosphatidylserine to the cell surface, that is, the outer side of the cell membrane. Exposure of phosphatidylserine on the cell surface promotes blood coagulation and inflammatory response. Annexin V, upon binding to phosphatidylserine externalized on the cell surface, can block the procoagulant and proinflammatory activity of phosphatidylserine. Annexin V labeled with the green fluorescent probe FITC, namely Annexin V-FITC, can be used for a very simple and direct detection of the externalization of phosphatidylserine, a key characteristic of apoptosis, using flow cytometry or fluorescence microscopy.

The cells were cultured in black-wall clear-bottom 96-well plates. Cells in the experimental group were transfected with EV-3C-mRNA (n=3; 100ng/well). After the apoptosis induction was completed, the cells were centrifuged with a centrifuge at 1000×g for 5 minutes. The cell medium was removed by pipetting, the cells were washed once with PBS, and centrifuged at 1000×g for 5 minutes, and then PBS was removed by pipetting. 195μL of Annexin V-FITC binding solution was added. 5μL of Annexin V-FITC was added and gently mixed. 10μL of propidium iodide staining solution was added and gently mixed. The cells were incubated at room temperature (20 to 25 °C) in the dark for 10 to 20 minutes, and then placed in an ice bath. It should be noted that aluminum foil should be used to protect the cells from light. The cells were observed immediately under a fluorescence microscope. Annexin V-FITC emitted green fluorescence, while propidium iodide (PI) emitted red fluorescence. Note: The cells should be examined immediately after staining, preferably within 1 hour.

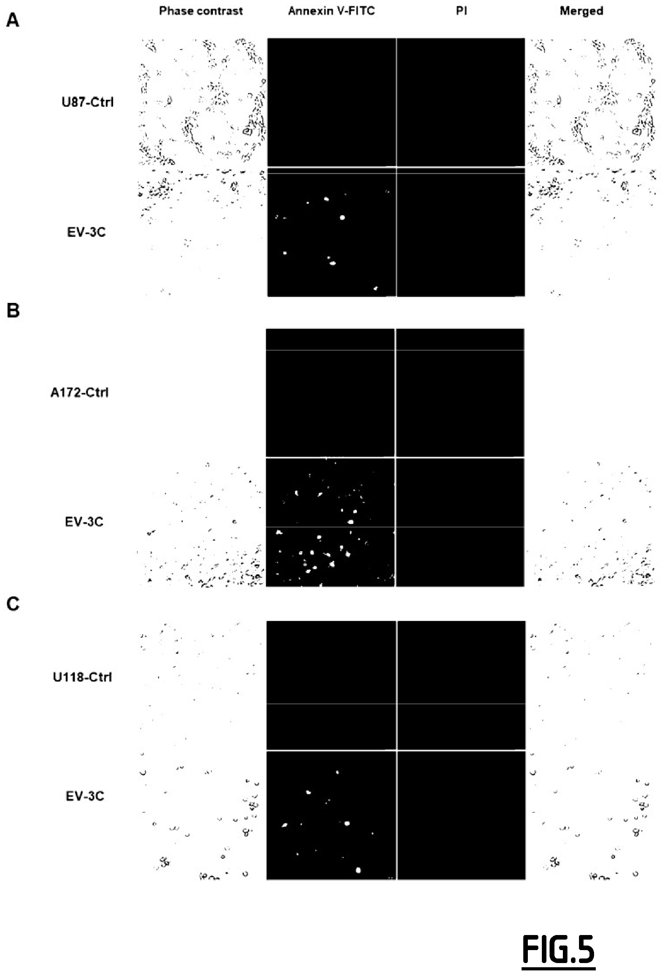

The staining results of Annexin V-FITC and propidium iodide (PI) were shown in Figure 5. Panels A to C showed the staining results of U87 MG, A172, and U118 MG cells, respectively. Cells stained with only green fluorescence represented apoptotic cells, cells stained with both green and red fluorescence represented necrotic cells, and cells without fluorescence staining were normal cells. In the Ctrl group of Panels A, B, and C, Annexin V-FITC and propidium iodide staining showed very weak signals, indicating that U87 MG, A172, and U118 MG cells remained in a normal state. In contrast, the 3C mRNA treatment group exhibited numbers of significant apoptosis and necrosis cells.

In summary, EV-3C-mRNA could promote apoptosis in various glioblastoma cells.

Example 6: Validation of activity of EV-3C on downregulation of MAPK signaling pathway-related proteins in tumor cells by Western Blot assay

This example was performed based on the EV-3C-mRNA prepared in Example 1.

Experimental materials: The human glioblastoma cells U87 MG used in the experiment were purchased from ATCC (Cat. No.: HTB-14), and A172 were purchased from ATCC (Cat. No.: CRL-1620). The above cells were cultured in a cell incubator at 37°C with 5% $CO_2$. The complete media used for cell growth were DMEM medium with high glucose (purchased from Gibco, Cat. No.: 11995065) and MEM medium (purchased from Gibco, Cat. No.: 10370021), which were supplemented with 10% fetal bovine serum (purchased from Gibco, Cat. No.: 16000044) and penicillin-streptomycin double antibiotics (purchased from Gibco, Cat. No.: 2321152). Horseradish peroxidase (HRP)-conjugated goat anti-mouse IgG antibody was purchased from Abcam (Cat. No. ab205719). Defat Dried Milk for blocking was purchased from APPLYGEN (Cat. No.: P1622), BCA Protein Assay Kit was purchased from APPLYGEN (Cat. No.: P1511), RIPA Lysis Buffer was purchased from APPLYGEN (Cat. No.: C1053), ECL Chemiluminescence Solution P1000 was purchased from APPLYGEN (Cat. No.: P1000-25), Enterovirus 71 3C Antibody (B3) was purchased from NB (Cat. No.: NBP3-13501), the Caspase-3 (D3R6Y) Rabbit mAb was purchased from CST (Cat. No.: #14220), hnRNP A1 (K350) Antibody was purchased from CST (Cat. No.: #4296), p38 MAPK Antibody was purchased from CST (Cat. No.: #9212), and PSF antibody (D-8) was purchased from Santa Cruz Biotechnology (sc-271796).

Preparation of reagents: SDS-PAGE gel electrophoresis buffer solution: 3.03 g of Tris base, 18.7 g of glycine, and 1 g of SDS were accurately weighed, and ultrapure water was added to a final volume of 1 L. TBST solution: 3 g of Tris base and 8 g of NaCl were accurately weighed, and ultrapure water was added to a final volume of 1 L, thereby to obtain TBS solution; TBST solution was then prepared by adding 0.1% Tween-20 to the TBS solution. WB blocking solution: 2.5 g of BSA was accurately weighed and added to 50 mL of TBST solution to obtain a blocking solution containing 5% BSA; 10× Transfer buffer: 30.3 g of Tris-base and 144 g of glycine were accurately weighed, and ultrapure water was added to a final volume of 1 L. 1× Transfer buffer: to 100 mL of 10× transfer buffer, 200 mL of methanol and 700 mL of ultrapure water was added. Separating gel: $H_2O$, 30% Acr-Bis (29:1) and separating gel buffer (pH 8.8) were mixed, followed by addition of 10% APS and TEMED with thorough mixing. Stacking gel: $H_2O$, 30% Acr-Bis (29:1) and stacking gel buffer (pH 6.8) were mixed, followed by addition of 10% APS and TEMED with thorough mixing.

The experimental scheme was as follows:

The MAPK pathway comprises four main branches: ERK, JNK, p38/MAPK and ERK5. Among them, JNK and p38 share similar functions and are involved in inflammation, apoptosis and cell growth; ERK is primarily responsible for cell growth and differentiation, and its upstream signal is the well-known Ras/Raf protein. The mitogen-activated protein kinase (MAPK) signaling pathway is an important intracellular signaling cascade, which is composed of Ser/Thr protein kinases widely expressed in cells, participates in a series of cell physiological activities such as cell growth, development, differentiation, and apoptosis, and is an associated factor in tumorigenesis. ERK and p38 converge at MNK1/2, and the downstream proteins of MNK1/2, such as PSF, eIF4E, hnRNPA1, cPLA2, Spry2, are all highly upregulated proteins in tumor cells.

The detection method of EV-3C down-regulating MAPK signaling pathway-related proteins in tumor cells was described as follows:

Cells were treated with EV-3C-mRNA, and a corresponding blank control was set up. After culturing the cells for 12 hours, the medium was discarded, the cells were washed once with PBS, and lysis solution was added to the 6-well plate at a ratio of 150 to 250 $\mu$L per well. The lysis solution was pipetted several times to ensure sufficient contact between the lysis buffer and the cells. After complete lysis, the mixture was centrifuged at 10,000 to 14,000 $\times$ g for 3 to 5 minutes. The supernatant was collected, and subsequent operations such as PAGE, Western blotting, and immunoprecipitation were performed. 3$\mu$L of the protein obtained in the previous step was pipetted and diluted 20-fold with 57$\mu$L of PBS, BCA Reagent and Cu Reagent (50:1) were mixed. 200$\mu$L of the BCA mixture was pipetted and added to a 96-well plate, 25$\mu$L of protein sample was added thereto, duplicate wells were set up, and then the plate was incubated at 37°C for 0.5h, followed by detection using a multi-functional microplate reader. The concentration of the protein to be tested was calculated based on the standard curve. After calculation, lysis solution was added to the corresponding protein to dilute it to the same concentration. Then, 5$\times$ Loading Buffer was added for subsequent sample loading.

The vertical electrophoresis glass plates were assembled. The separating gel and stacking gel for SDS-PAGE were prepared according to the formulations.

The prepared separating gel was added between the glass plates, up to 1.5 cm below the upper edge of the short plate. Ultrapure water was gently added to the upper edge of the short plate, and polymerization was carried out at room temperature for 25 to 30 minutes. After polymerization of the separating gel, the ultrapure water above the separating gel was slowly poured off, and carefully blotted dry with absorbent paper. The prepared stacking gel was added, and a suitable sample comb was inserted. Polymerization was carried out at room temperature for 25 to 30 min. After polymerization of the stacking gel, the Mini-PROTEAN vertical electrophoresis device was assembled. The freshly prepared 1$\times$ electrophoresis buffer was added to immerse the upper edge of the short plate in the inner tank. Then, 25$\mu$g of protein Marker and sample protein were carefully loaded into each well. The electrophoresis buffer was topped up, the electrophoresis device was checked, and the power supply was connected. Initially, the electrophoresis was performed at a constant voltage of 60 V. When the bromophenol blue dye migrated out of the stacking gel, the voltage was increased to 80 V for constant-voltage electrophoresis. The electrophoresis was stopped when the bromophenol blue dye reached the bottom of the separating gel.

The PVDF membrane was pre-activated by immersion in anhydrous methanol for 1 min, was then equilibrated by immersion in 1$\times$ transfer buffer together with the transfer sponge and thick/thin filter paper for 15 min. Meanwhile, the Mini Trans-Blot transfer tank was placed in an ice box for ice-bath cooling, and an appropriate amount of freshly prepared 1$\times$ transfer buffer was added for pre-cooling. After the electrophoresis was completed, the vertical electrophoresis device was disassembled, the glass plates were pried open with a gel scraper, and the gel was carefully transferred onto a pre-degassed sponge-thick filter paper-thin filter paper assembly. The upper surface of the gel was gently rolled with a glass rod to remove air bubbles, constructing the transfer sandwich. The prepared transfer sandwich structure (assembled in the order of filter paper, membrane, gel, and filter paper from anode to cathode) was placed into the Mini Trans-Blot transfer tank, and 1$\times$ transfer buffer was added to cover the transfer sandwich. The electrotransfer device was checked, and an appropriate current and transfer time were set, and the power supply was connected to start electrotransfer. The transfer conditions were 4°C, 300 mA, for 1.5 h. After the electrotransfer was completed, the power supply was turned off, and the transfer device was disassembled. The transfer sandwich was carefully disassembled, and the PVDF membrane was placed with the protein side facing up, marked and transferred to a membrane-washing box containing an appropriate amount of 1$\times$ TBST buffer for rinsing with shaking at room temperature for 5 min. The buffer in the membrane-washing box was poured out, an appropriate amount of freshly prepared blocking solution was added for blocking with shaking at room temperature for 1 h. After blocking, the membrane was rinsed with an appropriate amount of 1$\times$TBST buffer with shaking at room temperature 3 times for 5 min each time. The PVDF membrane was transferred to an incubation box containing an appropriate amount of primary antibody working solution diluted at 1:1000-2000 (dilution ratio refers to the antibody instructions), and incubated with shaking overnight (>12 h) at 4°C. After incubation with the primary antibody, the membrane was rinsed with an appropriate amount of 1$\times$TBST buffer with shaking at room temperature 3 times for 5 min each time. The PVDF membrane was transferred to an incubation box containing an appropriate amount of secondary antibody working solution diluted at 1:4000 (dilution ratio refers to the antibody instructions), and incubated with shaking at room temperature for 1 h. After incubation with the secondary antibody, the membrane was rinsed with an appropriate amount of 1$\times$TBST buffer with shaking at room temperature 3 times for 5 min each time. Then, the PVDF membrane was transferred to an ECL chemiluminescence incubation box. The transferred PVDF membrane was trimmed to an appropriate size and placed in the antibody incubation box. An appropriate amount of ECL chemiluminescence reagent was added onto the protein side of the PVDF membrane, and luminescence detection was performed using a ChemiDoc XRS+ imaging system.

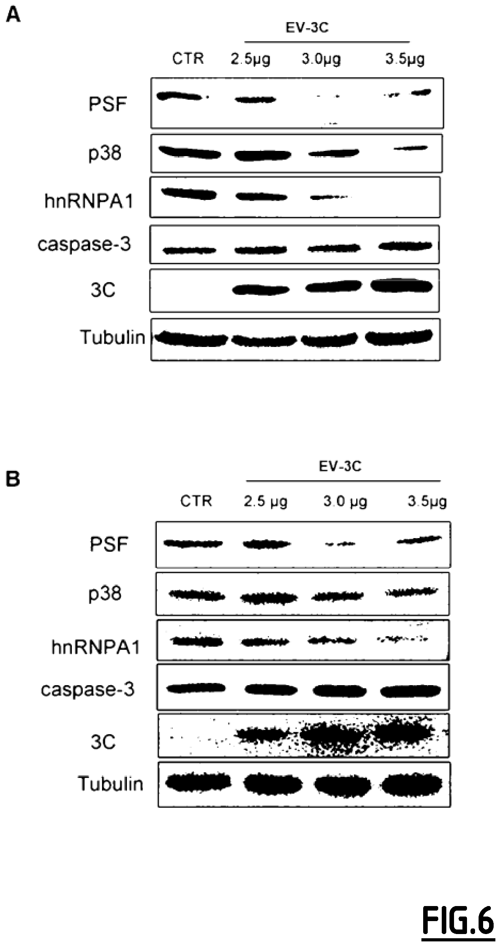

The experimental results were shown in Figure 6, in which Panel A showed the WB assay results in the U87 MG cell line, and Panel B showed the WB assay results in the A172 cell line. The results indicated that the EV-3C protein downregulated both the upstream p38 protein and downstream PSF [PTB (polypyrimidine tract-binding protein)-associated splicing factor] protein and hnRNPA1 in the MNK1/2 signaling pathway, and promoted the upregulation of the downstream apoptosis execution protein caspase-3 of hnRNPA1. Heterogeneous nuclear ribonucleoprotein (hnRNP) A1 is an RNA-binding protein that shuttles between the nucleus and the cytoplasm, and is involved in various RNA metabolic processes, such as pre-mRNA splicing and RNA transport. hnRNPA1 also is an IRES trans-acting factor (ITAF), which can bind to the IRES of apaf-1 to repress its translation, and thus inhibit apoptosis. Apaf-1, short for apoptotic protease activating factor-1), plays an important role in the mitochondrial-mediated apoptotic pathway. This protein assembles cytochrome c and deoxyadenosine triphosphate (dATP) to form an oligomeric apoptosome. The apoptosome exhibits its mature activation by binding to or cleaving of the procaspase-9. The activated protease-9 stimulates subsequent apoptotic proteases to control the cell apoptosis.

In summary, EV-3C broadly cleaved multiple key proteins in the MNK1/2 signaling pathway.

Example 7: The effect of EV-3C-mRNA on inhibition of cell viability of glioblastoma cells

This example was performed based on the EV-3C-mRNA prepared in Example 1.

Experimental materials: The human glioblastoma cells U87 MG used in the experiment were purchased from ATCC (Cat. No.: HTB-14), and A172 were purchased from ATCC (Cat. No.: CRL-1620). The above cells were cultured in a cell incubator at 37°C with 5% $CO_2$. The complete medium used for cell growth was DMEM medium with high glucose (purchased from Gibco, Cat. No.: 11995065) or MEM medium (purchased from Gibco, Cat. No.: 10370021), which were supplemented with 10% fetal bovine serum (purchased from Gibco, Cat. No.: 16000044) and penicillin-streptomycin double antibiotics (purchased from Gibco, Cat. No.: 2321152). Rupintrivirvr (AG7088) was purchased from MCE (Cat. No.: HY-106161). Cell-Titer Glo® Luminescent Cell Viability Assay Solution was purchased from Promega (Cat. No.: G7572). The microplate reader was purchased from Molecular Devices (model: SpectraMax M5).

The experimental scheme was as follows:

(1) Evaluation of the effect of EV-3C-mRNA on the viability of U87 MG and A172 cells

U87 MG cells and A172 cells were seeded in white-walled clear-bottom 96-well plates at a density of $1.0 \times 10^4$ per well, and cultured at 37°C with 5% $CO_2$ for 24 hours. Then, EV-3C-mRNA was diluted with Opti-MEM to final concentrations of 998.4 ng/ml, 499.2 ng/ml, 249.6 ng/ml, 124.8 ng/ml, 62.4 ng/ml, 31.2 ng/ml, and 15.6 ng/ml, and transfected into the cells using Lipo2000 liposomes. After 48 hours, the supernatant was discarded, and the diluted Cell-Titer Glo® Luminescent Cell Viability assay solution was added. The mixture was shaken in the dark for 5 minutes to lyse the cells, followed by standing for 3 minutes. Finally, the luminescence value of each well was measured using a microplate reader.

$$\text{Cell viability (\%)} = \text{average value of experimental group} / \text{average value of cell control group} \times 100$$

(2) Effect of Rupintrivir (AG7088) blocking the inhibition of U87 MG and A172 cell viability by EV-3C-mRNA

Rupintrivir (AG7088) is an effective, selective and irreversible 3C protease inhibitor. U87 MG cells and A172 cells were seeded in white-walled clear-bottom 96-well plates at a density of $1.0 \times 10^4$ per well, and cultured at 37°C with 5% $CO_2$ for 24 hours. Subsequently, EV-3C-mRNA was diluted with Opti-MEM to final concentrations of 998.4 ng/ml, 499.2 ng/ml, 249.6 ng/ml, 124.8 ng/ml, 62.4 ng/ml, 31.2 ng/ml, and 15.6 ng/ml, and transfected into cells with Lipo2000 liposomes. Compound AG7088 was serially diluted with 2% cell maintenance medium and then added to the 96-well plates to final concentrations of 10μM, 1μM and 0μM, respectively. After 48h, the supernatant was discarded, and the diluted Cell-Titer Glo® Luminescent Cell Viability assay solution was added. The mixture was shaken for 5min in the dark to lyse the cells, followed by standing for 3min. Finally, the luminescence value of each well was measured using a microplate reader.

$$\text{Cell viability (\%)} = \text{average value of experimental group} / \text{average value of cell control group} \times 100$$

Data analysis: The inhibition rate-concentration curve was fitted to a sigmoidal curve using Origin9.0 software to calculate the half-maximal effective concentration ($EC_{50}$) of AG7088 against 3C-mRNA. The half-cytotoxic concentration ($CC_{50}$) of the compound was calculated using the same method.

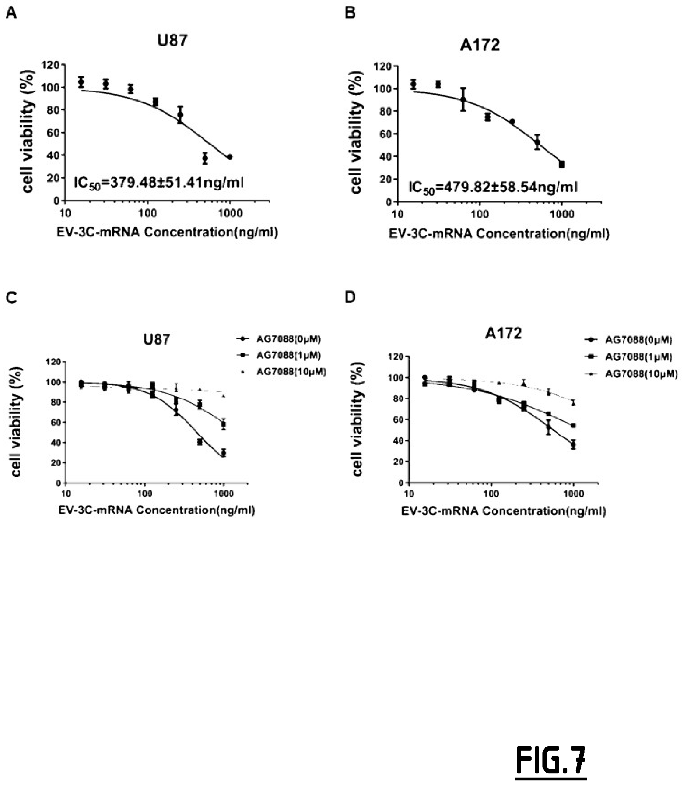

The experimental results were shown in Figure 7, Table 4 and Table 5. In Figure 7, Panel A showed the effect of EV-3C-mRNA on the viability of U87MG cells. From the results, it can be seen that the cell viability of U87 MG cells gradually decreased with the increase of 3C-mRNA transfection dose. Panel B showed the effect of EV-3C-mRNA on the viability of A172 cells. From the results, it can be seen that the cell viability of A172 cells gradually decreased with the increase of 3C-mRNA transfection dose. Figure C showed that AG7088 inhibited the decrease in cell viability of U87 MG cells caused by 3C-mRNA transfection, and inhibited the activity of 3C protein in U87 MG cells, with an $IC_{50}$ of 1.149 $\pm 0.19\mu$M. Meanwhile, the $CC_{50}$ value of AG7088 for U87 MG cells was >100$\mu$M. Panel D showed that AG7088 inhibited the decrease in cell viability of A172 cells caused by 3C-mRNA transfection, and inhibited the activity of 3C protein in A172 cells, with an $IC_{50}$ of 0.439$\pm$0.03$\mu$M. Meanwhile, the $CC_{50}$ value of AG7088 for A172 cells was >100$\mu$M.

In summary, the anti-tumor effect of EV-3C-mRNA depended on the activity of 3C protease.

Table 4. Inhibition of EV-3C-mRNA on viability of U87 MG and A172 cells

| Concentration (ng/ml) | 15.6 | 31.2 | 62.4 | 124.8 | 249.6 | 499.2 | 998.4 | 0 |
|---|---|---|---|---|---|---|---|---|
| Viability of U87 cells (%) | 104.62±6.18 | 102.92±5.66 | 98.38±5.24 | 87.32±4.46 | 75.63±10.29 | 37.27±6.69 | 38.38±1.56 | 100±3.27 |
| Viability of A172 cells (%) | 103.87±5.60 | 103.89±3.94 | 90.35±14.44 | 74.87±4.39 | 70.95±2.02 | 52.63±9.29 | 33.05±3.41 | 100±3.67 |

Table 5. Effect of Rupintrivir (AG7088) blocking the inhibition of U87 MG and A172 cell viability by EV-3C-mRNA

| | Concentration (ng/ml) | 15.6 | 31.2 | 62.4 | 124.8 | 249.6 | 499.2 | 998.4 |
|---|---|---|---|---|---|---|---|---|
| Viability of U87 cells (%) | AG7088 (10μM) | 94.66±1.37 | 93.94±2.55 | 91.93±2.91 | 96.77±1.12 | 94.40±3.25 | 92.86±1.39 | 86.90±1.28 |
| | AG7088 (1μM) | 97.72±1.71 | 95.64±2 | 94.60±2.46 | 96.14±2.80 | 81.93±2.42 | 77.50±3.43 | 58.11±4.46 |
| | AG7088 (0μM) | 99.97±0.49 | 98.59±1.32 | 96.36±3.53 | 87.32±2.44 | 72.63±4.62 | 40.59±2.05 | 29.71±3.03 |
| Viability of A172 cells (%) | AG7088 (10μM) | 96.86±1.08 | 96.14±3.29 | 94.23±3.01 | 95.27±0.90 | 95.44±2.39 | 86.12±2.56 | 76.13±2.14 |
| | AG7088 (1μM) | 93.52±1.09 | 93.21±1.59 | 92.78±2.46 | 78.70±2.71 | 73.51±2.84 | 65.51±1.11 | 54.32±0.91 |
| | AG7088 (0μM) | 100.29±1.06 | 97.19±3.14 | 88.01±1.23 | 78.87±0.72 | 72.62±3.22 | 52.63±5.53 | 36.28±3.43 |

Example 8: Encapsulation of EV-3C-mRNA in lipid nanoparticle (LNP)

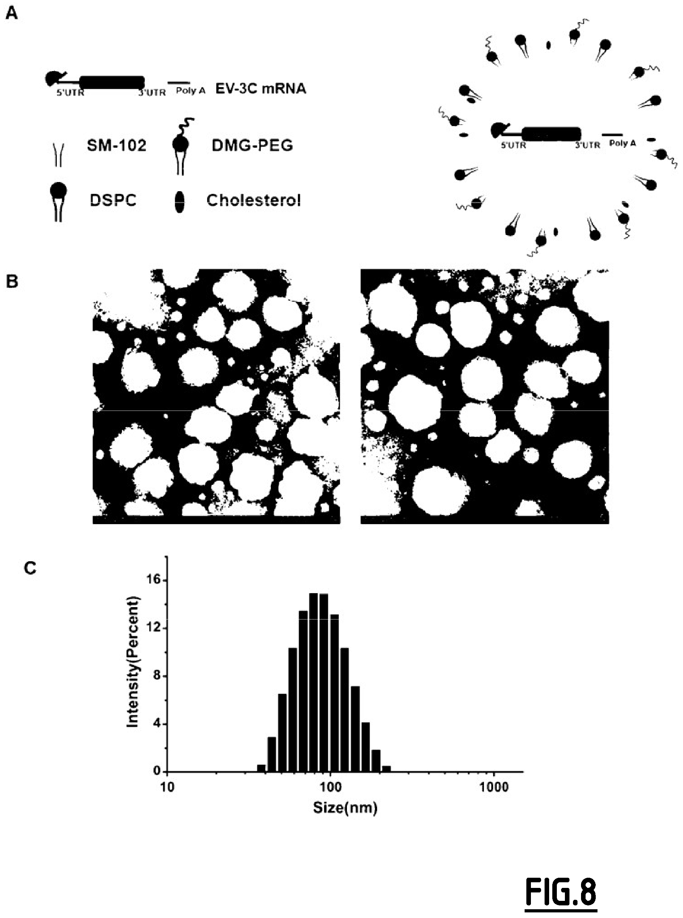

Experimental materials: SM-102 used in the experiment was purchased from MCE (Cat. No.: HY-134541), DSPC was purchased from Aladdin (Cat. No.: 816-94-4), Cholesterol was purchased from Sigma (Cat. No.: 57-88-5), PEG2000-DMG was purchased from MCE (Cat. No.: HY-145411), Sodium citrate buffer solution (RNase-free) was purchased from Thermo (Cat. No.: J63888.AP), Tris-HCl Buffer (RNase free, 1M, PH7.4) was purchased from Macklin Reagent (Cat. No.: 1185-53-1), Sucrose (≥99.5%, BioReagent) was purchased from Beyotime (Cat. No.: ST1672), sodium acetate solution (RNase free, PH 5.2, 3M) was purchased from Macklin Reagent (Cat. No. 6131-90-4), sodium hydroxide solution (RNase free, 2M) was purchased from Macklin Reagent (Cat. No. 1310-73-2), Acetic acid was purchased from Sigma (Cat. No. 64-19-7), and Quant-iT™ RiboGreen RNA Assay Kit was purchased from Thermo (Cat. No. R11490). The NanoAssemblr® Ignite was purchased from Precision Nanosystems (PNI) (Model: NIN0001), Zetasizer Ultra was purchased from Malvern Panalytical (Model: ZSU3305), and Fluoroskan™ FL Microplate Fluorometer and Luminometer was purchased from Thermo (Cat. No. 5200222).
The experimental scheme was as follows:
Lipids (ionizable cationic lipid SM-102, helper lipid DSPC, cholesterol, and PEGylated lipid PEG2000-DMG) were dissolved in ethanol at an optimal molar ratio. The LNP component formulation was SM-102: DSPC: Cholesterol: DMG-PEG = 50: 10: 38.5: 1.5. mRNA was diluted to the desired concentration in sodium citrate buffer solution. LNPs were generated by mixing lipids with mRNA using a microfluidic mixer. Subsequently, LNPs were dialyzed against Tris-HCl buffer with sucrose in a dialysis cassette. After dialysis, LNPs were filtered through a 0.22 μm filter and stored at -80°C. mRNA in LNPs was quantified using the RiboGreen method. Particle size and surface charge were determined by dynamic light scattering and zeta potential using a Zetasizer device. Endotoxin levels were detected using a dynamic colorimetric TAL method. LNP dispersion was prepared in 20 mM Tris, 87 mg/mL sucrose, 10.7 mM sodium acetate, pH 7.5.
The experimental results were shown in Figure 8 and Table 6. In Figure 8, Panel A showed the LNP components. The mRNA concentration detected by the RiboGreen assay was 120 μg/ml. Particle size parameters were measured using a Zetasizer Advance potentiometer. The diameter of the encapsulated LNP was 82.76 nm. The transmission electron microscopy results were shown in Panel B, and the particle size distribution was shown in Panel C.
Functional testing revealed that the average zeta potential of the LNPs carrying mRNA (LNP-mRNA) was -3.26 mV. The encapsulation efficiency of the LNPs carrying mRNA (LNP-mRNA) was 90.77%. The average endotoxin level of the LNPs carrying mRNA (LNP-mRNA) was less than 10 EU/ml.

Table 6. Particle size distribution of EV-3C-mRNA-LNP nanoparticles

| Intensity (Percent) | Size (d.nm) |
|---|---|
| 0.4 | 0 |
| 0.463 | 0 |
| 0.536 | 0 |
| 0.621 | 0 |
| 0.719 | 0 |
| 0.833 | 0 |
| 0.965 | 0 |
| 1.12 | 0 |
| 1.29 | 0 |
| 1.5 | 0 |
| 1.74 | 0 |
| 2.01 | 0 |
| 2.33 | 0 |
| 2.7 | 0 |
| 3.12 | 0 |
| 3.62 | 0 |
| 4.19 | 0 |
| 4.85 | 0 |

(continued)

| Intensity (Percent) | Size (d.nm) |
|---|---|
| 5.61 | 0 |
| 6.5 | 0 |
| 7.53 | 0 |
| 8.72 | 0 |
| 10.1 | 0 |
| 11.7 | 0 |
| 13.5 | 0 |
| 15.7 | 0 |
| 18.2 | 0 |
| 21 | 0 |
| 24.4 | 0 |
| 28.2 | 0 |
| 32.7 | 0 |
| 37.8 | 0.536 |
| 43.8 | 2.84 |
| 50.7 | 6.46 |
| 58.8 | 10.3 |
| 68.1 | 13.4 |
| 78.8 | 14.9 |
| 91.3 | 14.8 |
| 106 | 13.1 |
| 122 | 10.3 |
| 142 | 7.09 |
| 164 | 4.08 |
| 190 | 1.78 |
| 220 | 0.439 |
| 255 | 0 |
| 295 | 0 |
| 342 | 0 |
| 396 | 0 |
| 459 | 0 |
| 531 | 0 |
| 615 | 0 |
| 712 | 0 |
| 825 | 0 |
| 955 | 0 |
| 1110 | 0 |
| 1280 | 0 |
| 1480 | 0 |

(continued)

| Intensity (Percent) | Size (d.nm) |
|---|---|
| 1720 | 0 |
| 1990 | 0 |
| 2300 | 0 |
| 2670 | 0 |
| 3090 | 0 |
| 3580 | 0 |
| 4150 | 0 |
| 4800 | 0 |
| 5560 | 0 |
| 6440 | 0 |
| 7460 | 0 |
| 8630 | 0 |

Example 9: Evaluation of *in vivo* delivery capacity of lipid nanoparticles

Experimental materials: Female BALB/c nude mice (n=6, aged 6-8 weeks) were purchased from SPF (Beijing) Biotechnology Co., Ltd., with a clear source and qualified inspection. D-Luciferin potassium salt was purchased from Beyotime (Product No.: ST196-100mg); the *in vivo* imaging instrument was the PerkinElmer IVIS Spectrum CT, a small animal *in vivo* three-dimensional multimodal imaging system; FLuc mRNA-LNPs were purchased from VectorBuilder.

The experimental plan was as follows:

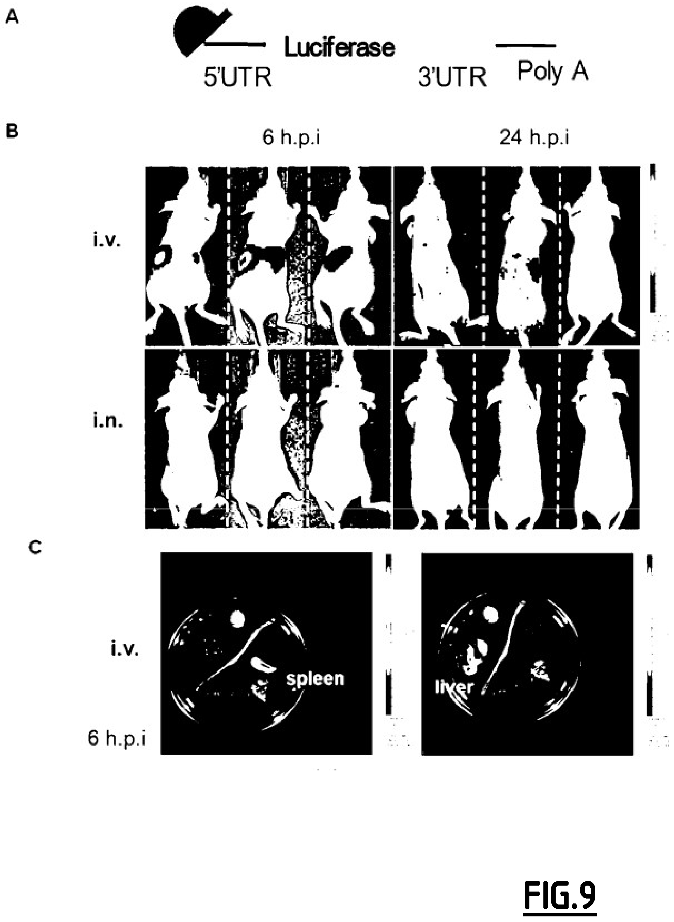

Prior to the *in vivo* experiment, it was necessary to evaluate the *in vivo* delivery capacity of nanoparticles and the *in vivo* protein expression capacity. First, it was necessary to construct luciferase reporter gene nanoparticles FLuc mRNA-LNPs (the structure of FLuc mRNA was shown in Panel A of Figure 9) to detect the *in vivo* distribution of mRNA-LNPs and the duration of *in vivo* potency maintenance of mRNA. Female BALB/c nude mice aged 6-8 weeks were inoculated with 12 μg of FLuc mRNA-LNP (120 μg/ml, i.e. 100 μL per mouse) via tail vein injection and intranasal instillation, respectively. IVIS imaging was performed at 6 h.p.i and 24 h.p.i., respectively. Before imaging, each mouse was intraperitoneally injected with 10 μL/g D-luciferin potassium salt. After 10 to 20 minutes of *in vivo* injection, imaging analysis was performed once the fluorescence signal reached the strongest stable plateau phase. After imaging of 3 mice of the intranasal instillation group and 3 mice of the tail vein injection group, one mouse with the strongest signal in each group was immediately dissected, and the brain and internal organs were collected and placed in culture dishes for imaging.

The experimental results were shown in Figure 9. For the tail vein injection group, the *in vivo* fluorescence intensity was the strongest at 6 h.p.i, and the signal was weak at 24 h.p.i, in contrast, almost no signal was detected in the intranasal instillation group by *in vivo* imaging, as shown in Panel B. One mouse from the tail vein injection group was dissected at 6 h.p.i, and it was found that the nanoparticles were almost concentrated in the spleen and liver, as shown in Panel C.

In summary, neither the tail vein injection nor the intranasal instillation can penetrate the blood-brain barrier to target brain tissue. Therefore, the optimal administration route for mice with *in situ* brain tumors was *in situ* brain delivery.

Example 10: Effect of EV-3C-mRNA-LNP inhibiting the growth of *in situ* brain tumors in mice and prolong survival time of mice

This example was performed based on the EV-3C-mRNA-LNP prepared in Example 8.

Experimental materials: Female BALB/c nude mice (n=30, aged 6-8 weeks) were purchased from SPF (Beijing) Biotechnology Co., Ltd., with a clear source and qualified inspection. U87 MG-luc (human glioma cell luc stable transformation strain) was purchased from Fenghui Biotechnology; D-Luciferin potassium salt was purchased from Beyotime (Product No.: ST196-100mg); the *in vivo* imaging instrument was the PerkinElmer IVIS Spectrum CT, a small animal *in vivo* three-dimensional multimodal imaging system; FLuc mRNA-LNPs was purchased from VectorBuilder; Hamilton microsyringe was purchased from the Shanghai Representative Office of Hamilton Bonatus AG, Switzerland. 27-G syringe needles were purchased from BD (Cat. No.: 20200520); glass slides were purchased from Servicebio; upright optical microscope was manufactured by Nikon, Japan (model: Nikon EclipseE100); imaging system was manufactured by Nikon, Japan (model: Nikon DS-U3); anhydrous ethanol was purchased from Sinopharm Chemical Reagent Co., Ltd. (Cat. No.: 100092683); xylene was purchased from Sinopharm Chemical Reagent Co., Ltd. (Cat. No.: 10023418); HE stain was purchased from Servicebio (Cat. No.: G1005); and differentiating solution was purchased from Servicebio (Cat. No.: G1005-3).

The experimental scheme was as follows:

(1) Establishment of *in situ* brain tumor model in mice and evaluation of *in vivo* efficacy of EV-3C-mRNA-LNP

1.1. Female BALB/c nude mice aged 6-8 weeks were anesthetized via intraperitoneal injection of 1% pentobarbital sodium (0.1 mL/10 g body weight).

1.2. After disinfecting the scalp with 75% ethanol, a vertical incision was made along the cranial midline to expose the bregma. The inoculation site was located 0 mm anterior to the bregma midpoint, 3 mm right lateral to the sagittal suture, and at a depth of 3 mm.

1.3. A microsyringe loaded with 5 $\mu$L of $1\times10^5$ U87 MG-luc cell suspension was inserted vertically into the striatum through a 27-G needle hole. The tumor cell suspension was slowly injected at a rate of 0.5 $\mu$L/min. The needle was kept in place for 2 minutes prior to being slowly withdrawn. The scalp was then sutured and the wound was disinfected with tincture of iodine.

1.4. Postoperatively, the mice were placed in a new cage, and placed on a 37°C electric heating blanket to maintain warmth until fully awake, after which the cage was returned to the rack.

1.5. The general conditions of the mice, including their mental state, diet, activity, and body weight, were observed daily. IVIS imaging was performed weekly.

1.6. After tumor implantation, the mice were divided into two groups for treatment: the two groups received *in situ* intracerebral injection of EV-3C mRNA-LNP once a week (1.2$\mu$g), and twice a week (2.4$\mu$g), respectively.

1.7. The survival time and body weight changes of the mice were recorded, and IVIS signals were monitored weekly.

(2) HE staining of different groups of *in situ* brain tumors

On the 21st day after tumor implantation, brain tissues were collected from the mice in the PBS group, the EV-3C mRNA-LNP (1.2$\mu$g) group, and the EV-3C mRNA-LNP (2.4$\mu$g) group, respectively, for HE staining and histological analysis.

2.1. Deparaffinization and rehydration of tissue sections: The sections were sequentially immersed in ethoxyethyl acetate I at 37°C for 6 hours, ethoxyethyl acetate II at 37°C overnight, ethoxyethyl acetate III at room temperature for 10-15 minutes, ethoxyethyl acetate IV at room temperature for 10-15 minutes, 100% ethanol I for 10 minutes, 100% ethanol II for 10 minutes, 95% ethanol for 10 minutes, 90% ethanol for 10 minutes, 80% ethanol for 10 minutes, followed by rinsing with tap water.

2.2. Hematoxylin staining: The sections were immersed in hematoxylin solution for 8-10 minutes, rinsed with tap water, differentiated with the differentiation solution, rinsed again with tap water, blued with the bluing solution, and finally rinsed with running water.

2.3. Eosin staining: The sections were sequentially dehydrated in 85% and 95% gradient alcohol for 5 minutes each, then stained in eosin solution for 8-10 minutes.

2.4. Dehydration and mounting: The sections were sequentially dehydrated in anhydrous ethanol I for 5 min, anhydrous ethanol II for 5 min, anhydrous ethanol III for 5 min, cleared in xylene I for 5 min, xylene II for 5 min, and mounted with neutral balsam.

2.5. Microscope examination, image acquisition and analysis: The nucleus appeared blue and the cytoplasm appeared red.

Data analysis: Statistical significance of survival curves was calculated using the Log-Rank test. Data were presented as the mean $\pm$ standard error of the mean (SEM). $p< 0.05$ was considered statistically significant.

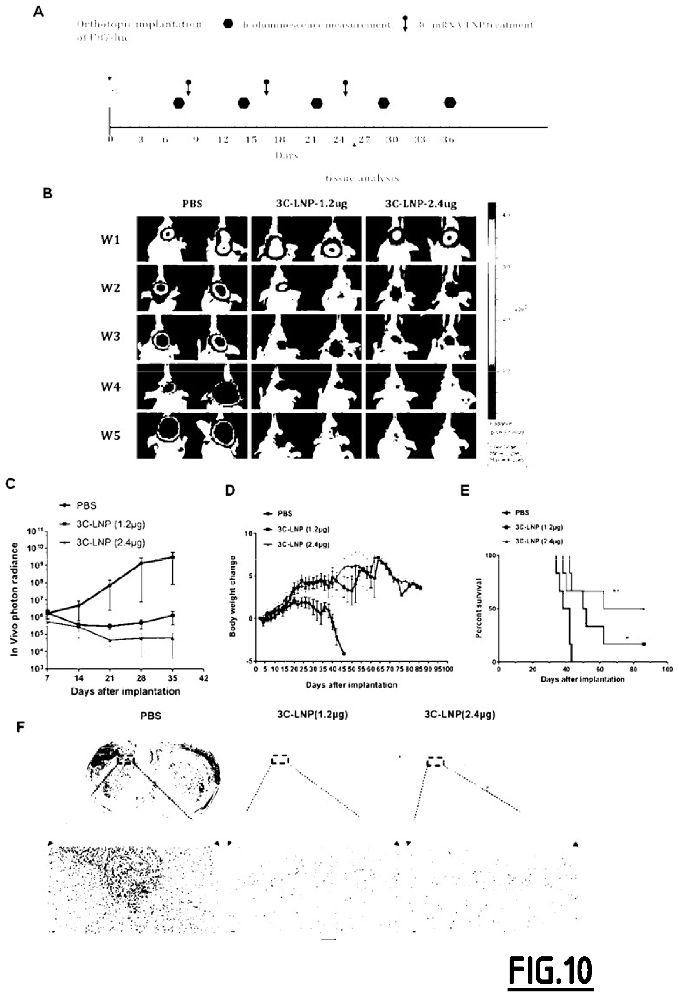

The experimental results were shown in Figure 10, and Tables 7 and 8. In Figure 10, Panel A showed the overall timeline of the *in vivo* experimental design. Panel B showed the *in vivo* imaging and quantitative fluorescence analysis results of the mice at Week 1 (W1), Week 2 (W2), Week 2 (W3), Week 4 (W4), and Week 5 (W5). The results of the PBS group showed that the *in situ* brain tumor model was successfully established, with a continuous increase of intracranial tumor signal. Compared with the PBS group, the signal in the once-weekly dosing group gradually decreased, whereas the signal in the twice-weekly dosing group decreased more significantly and even disappeared. Panel C showed the statistical analysis results of the signal intensity corresponding to Panel B. Figure D showed the statistical analysis of body weight changes. The survival curve in Panel E showed that the treatment groups had significantly prolonged survival time of mice. The HE staining results in Panel F showed that the tumors in the treatment groups were almost completely eliminated.

In summary, EV-3C-mRNA-LNP could efficiently inhibit the growth of *in situ* brain tumors in mice and prolong their survival time.

Table 7. Monitoring Results of IVIS signals in different groups of mice with *in situ* brain tumors

| Days after implantation | PBS | | | | | |
|---|---|---|---|---|---|---|
| 7 | 190000 | 220000 | 3200000 | 5200000 | 140000 | 820000 |
| 14 | 480000 | 38000 | 25000000 | 310000 | 200000 | 2100000 |
| 21 | 3600000 | 430000 | 4.1E+08 | 890000 | 1400000 | 5900000 |
| 28 | 17500000 | 1200000 | 7.9E+09 | 1700000 | 2600000 | 16500000 |
| 35 | 1.8E+08 | 29000000 | 1.7E+10 | 34000000 | 4000000 | 1.42E+08 |
| Days after implantation | 3C-LNP (1.2μg) | | | | | |
| 7 | 1100000 | 1100000 | 680000 | 550000 | 6500000 | 500000 |
| 14 | 530000 | 310000 | 97000 | 34000 | 760000 | 320000 |
| 21 | 240000 | 610000 | 190000 | 12000 | 590000 | 100000 |
| 28 | 1000000 | 1400000 | 160000 | 0 | 0 | 140000 |
| 35 | 5600000 | 1000000 | 690000 | 0 | 0 | 80000 |
| Days after implantation | 3C-LNP (2.4μg) | | | | | |
| 7 | 270000 | 110000 | 170000 | 160000 | 860000 | 1500000 |
| 14 | 43000 | 24000 | 42000 | 95000 | 89000 | 1300000 |
| 21 | 0 | 0 | 16000 | 140000 | 0 | 120000 |
| 28 | 0 | 0 | 15000 | 330000 | 0 | 10000 |
| 35 | 0 | 0 | 8700 | 340000 | 0 | 11000 |

Table 8. Body weight changes (g) in mice with *in situ* brain tumors after tumor implantation

| Days after tumor implantation | PBS | | | | | | 3C-LNP (1.2μg) | | | | | | 3C-LNP (2.4μg) | | | | | |
|---|---|---|---|---|---|---|---|---|---|---|---|---|---|---|---|---|---|---|
| 2 | 0 | 0 | 0 | 0 | 0 | 0 | 0 | 0 | 0 | 0 | 0 | 0 | 0 | 0 | 0 | 0 | 0 | 0 |
| 4 | 0.33 | 1.25 | -0.61 | -1.98 | 0.79 | -1.46 | 0.03 | 0.12 | -0.24 | -1.14 | -0.51 | -1.02 | 1.03 | 0.99 | 0.33 | 0.67 | -1.13 | -0.22 |
| 6 | 0.48 | 1.81 | -0.15 | -0.74 | 1.31 | 1.58 | 1.64 | 1.13 | -0.03 | -0.52 | -0.62 | -0.86 | 1.86 | 0.49 | 1 | 0.69 | 0 | 0.77 |
| 8 | 0.87 | 1.95 | 0.49 | -0.57 | 1.2 | -0.18 | 1.03 | 1.65 | -0.08 | 0.11 | -0.94 | -1.18 | 1.69 | 0.26 | 0.8 | -0.29 | -0.12 | 1.08 |
| 10 | 1.72 | 1.34 | 0.68 | -0.57 | 1.38 | 1.54 | 1.85 | 1.06 | -0.03 | 1.17 | -0.76 | -0.26 | 2.68 | -0.34 | 0.95 | 0.69 | 0.88 | 2.37 |
| 12 | 1.86 | 1.53 | 0.99 | -0.46 | 1.76 | 1.71 | 1.16 | 1.35 | 1.06 | 1.04 | -0.33 | -0.06 | 2.35 | 0.37 | 0.89 | 0.46 | 2.15 | 2.59 |
| 14 | 2.86 | 0.62 | 0.93 | -1.44 | 1.4 | 0.21 | 1.46 | 2.45 | 1.65 | 1.69 | -0.16 | 9.3 | 3.05 | 0.4 | 0.78 | 1.38 | 2.54 | 2.63 |
| 16 | 2.91 | 1.34 | 1.21 | -0.67 | 2.08 | 1.25 | 1.61 | 3.24 | 2.74 | 2.15 | 0.27 | 0.98 | 3.92 | 1.77 | 1.21 | 1.71 | 3.48 | 3.51 |
| 18 | 2.59 | 1.37 | 2.05 | -0.17 | 1.92 | 1.35 | 1.67 | 2.81 | 3.62 | 4.04 | 0.62 | 1.04 | 4.19 | 1.9 | 1.17 | 1.79 | 3.55 | 3.52 |
| 20 | 2.45 | 1.58 | 3.28 | 0.61 | 2.18 | 2.57 | 2.37 | 5.02 | 5.17 | 5.11 | 1.76 | 1.6 | 5.76 | 3.39 | 1.83 | 2.19 | 4.65 | 3.85 |
| 22 | 1.63 | 1.93 | 2.51 | -0.06 | 2.92 | 1.81 | 2.5 | 5.13 | 5.19 | 5.64 | 2.02 | 1.69 | 5.76 | 2.68 | 1.94 | 2.28 | 4.19 | 4.06 |
| 24 | 1.92 | 1.71 | 2.62 | -0.32 | 3.52 | 2.07 | 2.88 | 6.09 | 5.72 | 6.33 | 3.49 | 1.99 | 6.38 | 3.69 | 2.3 | 2.43 | 3.75 | 4.55 |
| 26 | 2.43 | 1.48 | 2.71 | -1.05 | 3.72 | 2.07 | 2.56 | 6.15 | 5.11 | 5.65 | 3.2 | 2.13 | 6.41 | 2.85 | 2.38 | 2.46 | 3.79 | 4.13 |
| 28 | 2.71 | 1.36 | 2.35 | -1.12 | 3.61 | 1.88 | 2.51 | 6.55 | 5.37 | 5.21 | 3.04 | 2.47 | 6.81 | 3.12 | 2.32 | 2.99 | 4.06 | 4.38 |
| 30 | 2.66 | 1.15 | 1.75 | -2.76 | 3.52 | 1.67 | 2.47 | 6.47 | 4.87 | 5.22 | 3.09 | 2.97 | 6.37 | 3.29 | 2.36 | 3.15 | 4.17 | 4.98 |
| 32 | 2.54 | 1.13 | 1.43 | -4.68 | 3.4 | 1.57 | 2.51 | 7.02 | 5 | 5.26 | 3.4 | 2.61 | 6.3 | 4.24 | 2.35 | 3.91 | 4.18 | 5.04 |
| 34 | 1.46 | 1.03 | 1.38 | -5.62 | 3.41 | 1.52 | 2.17 | 7.14 | 6.04 | 5.26 | 3.79 | 2.62 | 6.38 | 4.49 | 1.78 | 4.1 | 4.47 | 5.12 |
| 36 | -0.34 | 1.88 | 1.29 |  | 3.26 | 1.51 | 1.29 | 5.49 | 6.09 | 5.95 | 3.73 | 2.28 | 6.56 | 4.69 | 1.68 | 4.25 | 4.57 | 5.27 |
| 38 |  | 1.63 | 0.53 |  | 3.16 | -0.8 | -1.76 | 3.95 | 7.17 | 6.91 | 4.47 | 1.73 | 7.49 | 5.04 | 1.14 | 4.82 | 2.45 | 5.47 |
| 40 |  | 1.16 |  |  | 0.85 | -2.32 |  | 2.92 | 7.35 | 7.14 | 5.25 | 1.29 | 8.13 | 5.51 | -1.18 | 5.02 | 0.88 | 5.93 |
| 42 |  | -2.88 |  |  | -0.2 | -3.44 |  | 2.13 | 6.63 | 3.4 | 5.38 |  | 8.57 | 5.59 |  | 5.49 | -0.54 | 5.09 |
| 46 |  | -4.15 |  |  |  |  |  | 1.02 | 5.83 | 2.37 | 5.5 |  | 8.74 | 6.06 |  | 5.63 |  | 3.53 |
| 48 |  |  |  |  |  |  |  | 3.23 | 6.07 | 1.72 | 5.38 |  | 9.92 | 5.58 |  | 5.98 |  | 3.2 |
| 50 |  |  |  |  |  |  |  | 0.14 | 6.63 | 0.49 | 5.76 |  | 9.63 | 5.91 |  | 6.25 |  | 2.45 |
| 52 |  |  |  |  |  |  |  | 6.53 | -0.22 | 5.8 |  |  | 9.89 | 6.32 |  | 6.49 |  | 2.1 |
| 54 |  |  |  |  |  |  |  | 5 |  | 5.96 |  |  | 9.94 | 6.48 |  | 6.58 |  | 1.71 |

| Days after tumor implantation | PBS | | | | | | | | 3C-LNP (1.2µg) | | | | 3C-LNP (2.4µg) | | | | | |
|---|---|---|---|---|---|---|---|---|---|---|---|---|---|---|---|---|---|---|
| 56 | | | | | | | | 5.52 | | 6 | | 10.1 1 | 5.23 | | 6.19 | | J.98 |
| 58 | | | | | | | | 4.67 | | 6.1 | | 8.43 | 5.99 | | 6.3 | | -0.13 |
| 60 | | | | | | | | 3.63 | | 6.12 | | 8.44 | 6.24 | | 7.58 | | -0.2 |
| 62 | | | | | | | | 2.45 | | 7.02 | | 7.51 | 7.38 | | 6.76 | | |
| 64 | | | | | | | | | | 7.12 | | 7.42 | 6.68 | | 6.86 | | |
| 66 | | | | | | | | | | 6.46 | | 7.19 | 6.9 | | 6.22 | | |
| 68 | | | | | | | | | | 6.4 | | 6.38 | 5.46 | | 6.09 | | |
| 70 | | | | | | | | | | 5.22 | | 5.94 | 5.36 | | 6.03 | | |
| 72 | | | | | | | | | | 4.77 | | 4.56 | 3.43 | | 5.26 | | |
| 74 | | | | | | | | | | 4.44 | | 4.64 | 3.79 | | 5.41 | | |
| 76 | | | | | | | | | | 2.78 | | 4.64 | 3.04 | | 5.2 | | |
| 78 | | | | | | | | | | 3.27 | | 3.97 | 4.41 | | 4.91 | | |
| 80 | | | | | | | | | | 3.77 | | 3.58 | 4.16 | | 4.61 | | |
| 82 | | | | | | | | | | 4.04 | | 4.13 | 4.09 | | 4.75 | | |
| 84 | | | | | | | | | | 3.74 | | 4.25 | 3.21 | | 4.05 | | |
| 86 | | | | | | | | | | 3.57 | | 4.18 | 2.99 | | 4.15 | | |

(3) Evaluation of *in vivo* safety of EV-3C-mRNA-LNP

Experimental materials: Female BALB/c nude mice (n=6, aged 6-8 weeks) were purchased from SPF (Beijing) Biotechnology Co., Ltd., with a clear source and qualified inspection. Hamilton microsyringes were purchased from the Shanghai Representative Office of Hamilton Bonatus AG, Switzerland; 27-G syringe needles were purchased from BD (Cat. No.: 20200520); glass slides were purchased from Servicebio; upright optical microscope was manufactured by Nikon, Japan (model: Nikon EclipseE100); imaging system was manufactured by Nikon, Japan (model: Nikon DS-U3); anhydrous ethanol was purchased from Sinopharm Chemical Reagent Co., Ltd. (Cat. No.: 100092683); xylene was purchased from Sinopharm Chemical Reagent Co., Ltd. (Cat. No.: 10023418); HE stain was purchased from Servicebio (Cat. No.: G1005); differentiating solution was purchased from Servicebio (Cat. No.: G1005-3).

The experimental scheme was as follows:

Parallel to the *in vivo* efficacy evaluation experiment of EV-3C-mRNA-LNP, an *in vivo* safety evaluation experiment was performed in female BALB/c nude mice aged 6-8 weeks without *in situ* tumor implantation, which received *in situ* intracerebral injection of EV-3C mRNA-LNP once a week twice a week, with the same number of injections as the treatment group of the brain tumor model. The mouse's body weight was recorded throughout the study. After one month of observation, three mice per group were sacrificed, and tissues including the heart, liver, spleen, lung, kidney, and brain were collected for pathological section analysis.

1. Deparaffinization and rehydration of tissue sections: The sections were sequentially immersed in ethoxyethyl acetate I at 37°C for 6 hours, ethoxyethyl acetate II at 37°C overnight, ethoxyethyl acetate III at room temperature for 10-15 minutes, ethoxyethyl acetate IV at room temperature for 10-15 minutes, 100% ethanol I for 10 minutes, 100% ethanol II for 10 minutes, 95% ethanol for 10 minutes, 90% ethanol for 10 minutes, 80% ethanol for 10 minutes, followed by rinsing with tap water.

2. Hematoxylin staining: The sections were immersed in hematoxylin solution for 8-10 minutes, rinsed with tap water, differentiated with the differentiating solution, rinsed again with tap water, blued with the bluing solution, and finally rinsed with running water.

3. Eosin staining: The sections were sequentially dehydrated in 85% and 95% gradient alcohol for 5 minutes each, then stained in eosin solution for 8-10 minutes.

4. Dehydration and mounting: The sections were sequentially dehydrated in anhydrous ethanol I for 5 min, anhydrous ethanol II for 5 min, anhydrous ethanol III for 5 min, cleared in xylene I for 5 min, xylene II for 5 min, and mounted with neutral balsam.

5. Microscope examination, image acquisition and analysis: The nucleus appeared blue and the cytoplasm appeared red.

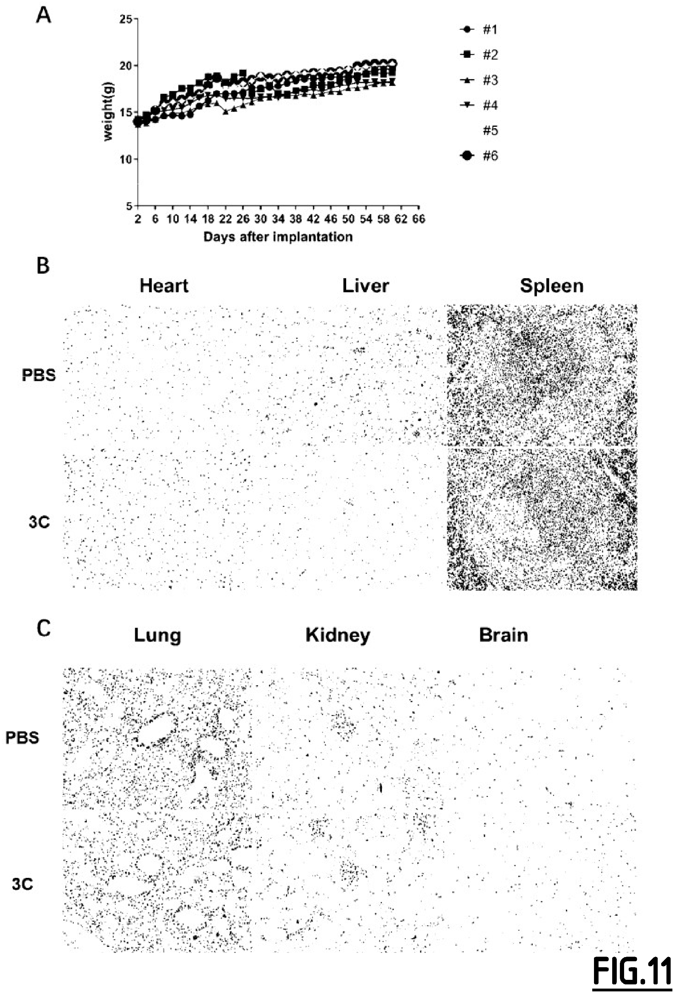

The results were shown in Figure 11. Panel A showed the trend of body weight changes, indicating that the mice's body weight exhibited a steady increasing trend without any decrease. Panels B and C showed comparisons of tissue sections of mice between the PBS group and the 3C group.

For heart tissue, compared with the PBS group, no obvious abnormalities were observed in the myocardial fibers in the 3C group. The myocardial fibers in the field of view were evenly stained, with clear cell boundaries and consistent orientation. Additionally, the cross-striations of cardiomyocytes were distinct with alternating light and dark bands, and no abnormalities were observed in the interstitium. No significant inflammatory cell infiltration was observed. For liver tissue, compared with the PBS group, the 3C group exhibited extensive granular degeneration of hepatocytes with loose cytoplasm and eosinophilic granules. Extramedullary hematopoietic foci were occasionally observed in the hepatic lobules, and no significant inflammatory cell infiltration was observed. For spleen tissue, compared with the PBS group, the 3C group showed a small number of extramedullary hematopoietic cells in the red pulp within the field of view. The splenic nodules had unclear boundaries with a few scattered granulocytes in the red pulp, and no obvious abnormalities were observed. For lung tissue, in the PBS group, mild thickening of the alveolar walls, a small amount of granulocyte infiltration in the alveolar walls, and local hemorrhagic foci with increased red blood cells in the alveolar spaces were observed in some areas, while no obvious abnormalities were observed in the bronchial structure. Compared with the PBS group, the 3C group exhibited extensive mild thickening of the alveolar wall, a small amount of granulocyte infiltration in the alveolar walls, with no obvious abnormalities in the bronchial structure, and no obvious hemorrhage. For kidney tissue, compared with the PBS group, the 3C group showed no obvious abnormalities in renal tissue; the glomeruli were evenly distributed with uniform cell number and matrix in the field of view. The renal tubular epithelial cells were round and plump with neatly arranged brush borders, and there was no obvious interstitial hyperplasia or inflammatory cell infiltration. For brain tissue, compared with the PBS group, the 3C group exhibited no significant difference. The neurons in the field of view were abundant, evenly distributed, and morphologically regular, with no obvious gliosis.

In summary, EV-3C-mRNA-LNP administered via *in situ* intracerebral injection demonstrated excellent *in vivo* safety.

Table 9: Body weight changes (g) of mice in the safety evaluation group after *in situ* intracerebral administration in mice

| Days after tumor implantation | #1 | #2 | #3 | #4 | #5 | #6 |
|---|---|---|---|---|---|---|
| 2 | 14 | 14.25 | 13.69 | 14.04 | 13.79 | 14.03 |
| 4 | 14.13 | 14.74 | 13.87 | 14.61 | 14.61 | 14.25 |
| 6 | 14.26 | 15.16 | 14.24 | 15.01 | 15.36 | 15.22 |
| 8 | 14.63 | 16.58 | 14.64 | 15.16 | 15.8 | 15.98 |
| 10 | 14.68 | 16.9 | 14.87 | 15.3 | 16.11 | 16.13 |
| 12 | 14.61 | 17.53 | 14.92 | 15.46 | 16.22 | 16.37 |
| 14 | 14.76 | 17.6 | 15.21 | 15.98 | 16.45 | 16.78 |
| 16 | 15.68 | 18.16 | 15.63 | 16.53 | 16.88 | 16.95 |
| 18 | 16.26 | 18.8 | 15.91 | 16.8 | 17.36 | 17.86 |
| 20 | 16.99 | 18.92 | 16 | 16.88 | 17.73 | 18.69 |
| 22 | 16.97 | 18.23 | 15.1 | 16.45 | 17.69 | 17.97 |
| 24 | 17 | 18.79 | 15.45 | 16.54 | 17.77 | 18.12 |
| 26 | 17.14 | 19.21 | 15.79 | 16.52 | 18.07 | 18.24 |
| 28 | 17.64 | 17.03 | 16.13 | 16.46 | 18.36 | 18.49 |
| 30 | 17.62 | 17.46 | 16.53 | 16.65 | 18.96 | 18.8 |
| 32 | 17.68 | 17.83 | 16.61 | 16.69 | 18.35 | 18.65 |
| 34 | 17.83 | 17.02 | 16.82 | 16.53 | 18.77 | 18.67 |
| 36 | 18.12 | 16.82 | 16.87 | 16.94 | 18.91 | 18.95 |
| 38 | 18.37 | 17.34 | 16.77 | 17.23 | 19.09 | 18.89 |
| 40 | 18.57 | 17.51 | 16.98 | 17.34 | 19.19 | 19.05 |
| 42 | 18.6 | 17.87 | 16.84 | 17.41 | 19.18 | 19.23 |
| 44 | 18.63 | 17.92 | 17.03 | 17.24 | 19.42 | 19.19 |
| 46 | 18.78 | 17.96 | 17.23 | 17.59 | 19.22 | 19.32 |
| 48 | 18.75 | 18.38 | 17.19 | 17.88 | 19.46 | 19.39 |
| 50 | 18.83 | 18.54 | 17.57 | 17.96 | 19.66 | 19.49 |
| 52 | 18.92 | 18.89 | 17.61 | 18.21 | 19.76 | 19.96 |
| 54 | 19.19 | 19.08 | 17.79 | 18.18 | 19.88 | 20.09 |
| 56 | 19.54 | 19.23 | 18.05 | 18.27 | 19.98 | 20.19 |
| 58 | 19.61 | 19.11 | 18.14 | 18.22 | 20.07 | 20.18 |
| 60 | 19.63 | 19.23 | 18.21 | 18.34 | 20.18 | 20.22 |

Example 11: Effect of EV-3C-mRNA on cell viability of other tumor cell lines

This example was performed based on the EV-3C-mRNA prepared in Example 1.

Experimental materials: The experimental materials in this Example were the same as above. Additional required experimental materials were as follows: Human umbilical vein endothelial cells (HUVEC) were purchased from Zhejiang Meisen Cell Technology Co., Ltd. (Cat. No.: CTCC-0804-PC), U2OS cells were purchased from ATCC (Cat. No.: HTB-96), CHL-1 cells were purchased from ATCC (Cat. No.: CRL-9446), DU145 cells were purchased from ATCC (Cat. No.: HTB-81), HCT-116 cells were purchased from ATCC (Cat. No.: CCL-247), Hela cells were purchased from ATCC (Cat. No.: CCL-2), HepG2 cells were purchased from ATCC (Cat. No.: HB-8065), AGS cells were purchased from ATCC (Cat. No.: CRL-1739), A549 cells were purchased from ATCC (Cat. No.: CCL-185), ACHN cells were purchased from ATCC (Cat. No.: CRL-1611), and Huh7 cells were purchased from the Chinese National Infrastructure of Cell Line Resource. The above cells were cultured in a cell incubator at 37°C with 5% $CO_2$. The complete medium used for cell growth was DMEM medium with high glucose (purchased from Gibco, Cat. No.: 11995065) or MEM medium (purchased from Gibco, Cat. No.: 10370021), which were supplemented with 10% fetal bovine serum (purchased from Gibco, Cat. No.: 16000044) and penicillin-streptomycin double antibiotics (purchased from Gibco, Cat. No.: 2321152). The endothelial cell medium was purchased from Zhejiang Meisen Cell Technology Co., Ltd. (Cat. No.: M22SN3004). Cell-Titer Glo® Luminescent Cell Viability Assay Solution was purchased from Promega (Cat. No.: G7572). The microplate reader was purchased from Molecular Devices (model: SpectraMax M5).

The experimental scheme was as follows:

HUVEC cells, A375 cells, U2OS cells, CHL-1 cells, DU145 cells, HCT-116 cells, Hela cells, HepG2 cells, AGS cells, A549 cells, ACHN cells and Huh7 cells were seeded in white-walled clear-bottom 96-well plates at a density of $1.0 \times 10^4$ per well and cultured at 37°C with 5% $CO_2$ for 24 hours. Subsequently, EV-3C-mRNA was diluted with Opti-MEM to final concentrations of 998.4 ng/ml, 499.2 ng/ml, 249.6 ng/ml, 124.8 ng/ml, 62.4 ng/ml, 31.2 ng/ml, and 15.6 ng/ml, respectively, and transfected into the cells using Lipo2000 liposomes. The final concentrations of EV-3C-mRNA transfected into HUVEC cells were 10000 ng/ml, 5000 ng/ml, 2500 ng/ml, 1250 ng/ml, 625 ng/ml, 312.5 ng/ml, 156.25 ng/ml, 78.125 ng/ml, 39.06 ng/ml, respectively. After 48 hours, the supernatant was discarded, and a diluted Cell-Titer Glo® Luminescent Cell Viability Assay solution was added. The mixture was shaken for 5 minutes in the dark to lyse the cells, followed by standing for 3 minutes. Finally, the luminescence value of each well was measured using a microplate reader.

$$\text{Cell viability (\%)} = \text{average value of experimental group/average value of cell control group} \times 100$$

The inhibition rate-concentration curve was fitted to a sigmoidal curve using Origin 9.0 software to calculate the half-maximal effective concentration ($EC_{50}$) of EV-3C-mRNA on the cells.

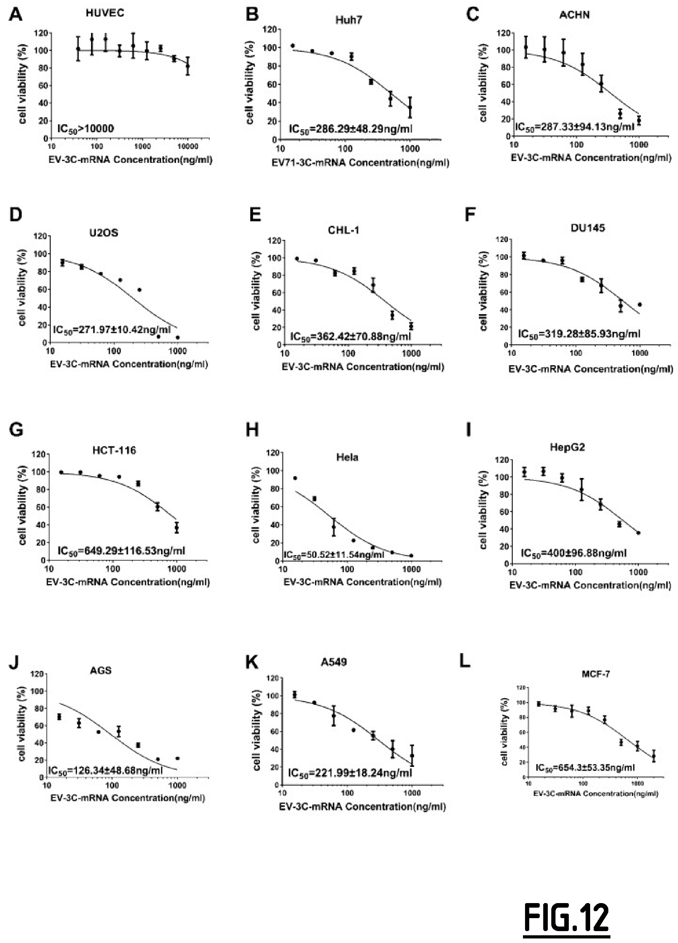

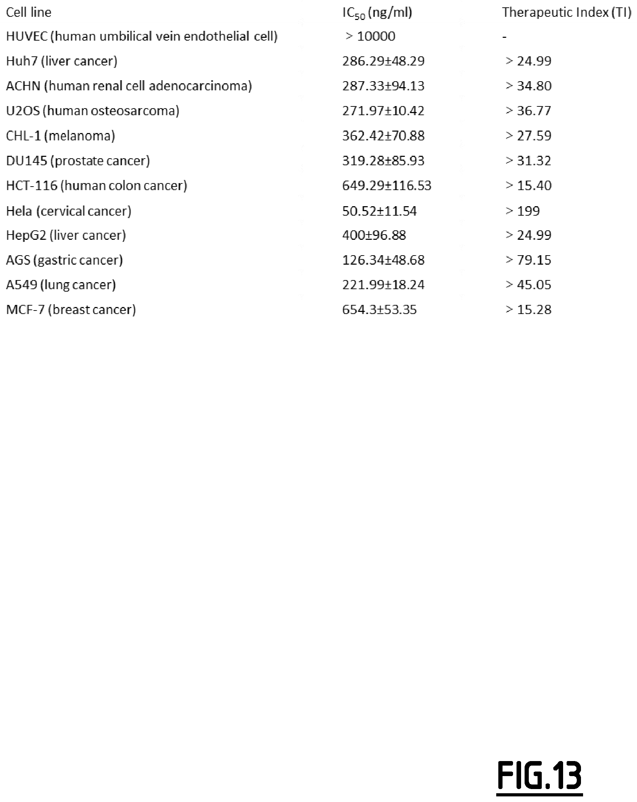

The results were shown in Figures 12 to 13 and Tables 10 to 11. EV-3C-mRNA exhibited no significant inhibitory effect on normal HUVEC cells, even at the highest concentration of 10000ng/ml, indicating that EV-3C-mRNA had excellent safety. Meanwhile, the results in Table 11 and Figure 12 demonstrated that EV-3C-mRNA could effectively inhibit the cell viability of a variety of human tumor cell lines, exhibiting broad-spectrum anti-tumor activity. Figure 13 showed a statistical summary of the $IC_{50}$ inhibition rates and therapeutic indices of EV-3C-mRNA against different cell lines.

In summary, EV-3C-mRNA exhibited broad-spectrum anti-tumor activity *in vitro*.

Table 10. Effect of EV-3C-mRNA on cell viability of normal HUVEC cells

| Concentration (ng/ml) | 39.06 | 78.125 | 156.25 | 312.5 | 625 | 1250 | 2500 | 5000 | 10000 | 0 |
|---|---|---|---|---|---|---|---|---|---|---|
| Cell viability of HUVEC (%) | 101.80 ±11.08 | 112.36 ±14.47 | 112.63 ±11.18 | 99.31 ±5.44 | 105.15 ±11.54 | 99.47 ±8.40 | 102.31 ±2.75 | 90.47 ±2.77 | 81.91 ±8.22 | 100 |

Table 11. Evaluation of EV-3C-mRNA on cell viability of other tumor cell lines

| Concentration (ng/ml) | 15.6 | 31.2 | 62.4 | 124.8 | 249.6 | 499.2 | 998.4 | 0 |
|---|---|---|---|---|---|---|---|---|
| Cell viability of Huh7 (%) | 101.96± 3.18 | 96.27 ±3.12 | 93.85 ±1.86 | 90.07 ±5.61 | 62.53 ±3.29 | 44.19 ±10.96 | 34.75 ±15.34 | 100 ±13.88 |
| Cell viability of ACHN (%) | 103.38± 17.86 | 100.63± 20.50 | 96.94 ±21.92 | 83.24 ±18.40 | 60.90 ±13.78 | 25.83 ±7.35 | 18.24 ±6.78 | 100 ±18.51 |
| Cell viability of U2OS (%) | 89.81 ±4.95 | 84.99 ±3.56 | 77.57 ±1.07 | 70.30 ±1.94 | 59.32 ±2.84 | 6.56 ±0.50 | 5.68 ±2.51 | 100 ±4.47 |
| Cell viability of CHL-1 (%) | 99.21 ±0.88 | 97.12 ±0.37 | 82.33 ±4.39 | 84.83 ±4.99 | 68.71 ±11.60 | 33.98 ±6.59 | 21.15 ±5.58 | 100 ±0.49 |
| Cell viability of DU145 (%) | 101.53± 5.31 | 95.83 ±5.31 | 95.92 ±5.41 | 74.26 ±3.44 | 67.39 ±11.07 | 44.32 ±9.55 | 45.79 ±3.01 | 100 ±6.04 |
| Cell viability of HCT-116 (%) | 99.50 ±2.52 | 99.55 ±2.53 | 95.71 ±0.51 | 94.39 ±3.05 | 86.67 ±3.89 | 60.51 ±6.47 | 36.80 ±8.10 | 100 ±4.39 |
| Cell viability of Hela (%) | 91.84 ±1.66 | 68.87 ±3.09 | 37.46 ±13.67 | 22.69 ±2.40 | 14.53 ±1.95 | 9.49 ±0.69 | 5.92 ±1.12 | 100 ±12.36 |
| Cell viability of HepG2 (%) | 105.83± 7.23 | 106.48± 6.61 | 99.03 ±6.97 | 85.55 ±17.64 | 68.44 ±8.95 | 45.58 ±4.27 | 35.52 ±2.04 | 100 ±0.60 |
| Cell viability of AGS (%) | 70.20 ±4.32 | 62.88 ±7.45 | 52.34 ±1.10 | 53.17 ±8.31 | 37.01 ±3.32 | 20.95 ±2.78 | 21.98 ±1.29 | 100 ±5.68 |
| Cell viability of A549 (%) | 101.21± 4.98 | 92.30 ±1.25 | 77.48 ±15.42 | 61.49 ±0.90 | 55.34 ±6.65 | 40.06 ±13.39 | 32.74 ±16.49 | 100 ±3.49 |
| Cell viability of MCF-7 (%) | 91.38±3 .09 | 88.23±6 .44 | 88.58±4 .08 | 76.76±4 .07 | 46.84±3 .42 | 41.51±5 .20 | 28.29±6 .28 | 100±0.3 7 |

Although the specific embodiments of the present application have been described in detail, it should be understood by those skilled in the art that various modifications and changes can be made to the details according to all the disclosed teachings, and these changes are within the scope of protection of the present application. The entire application is given by the attached claims and any equivalents thereof.

**Claims**

1. Use of a 3C protease of picornavirus or a nucleic acid molecule encoding the 3C protease in the preparation of a medicament for the prevention and/or treatment of a tumor in a subject.

2. The use according to claim 1, wherein the picornavirus is an enterovirus, such as a human enterovirus;

   preferably, the picornavirus is selected from the group consisting of novel enterovirus, poliovirus, coxsackievirus, rhinovirus and echovirus;
   preferably, the picornavirus is a novel enterovirus (e.g., *Enterovirus 68* (EV68), *Enterovirus 69* (EV69), *Enterovirus 70* (EV70), *Enterovirus 71* (EV71)), a coxsackievirus (e.g., *Coxsackievirus A14, Coxsackievirus A16, Coxsackievirus A6, Coxsackievirus B3)* or a rhinovirus (e.g., *Rhinovirus 2, Rhinovirus 7, Rhinovirus 25);*
   preferably, the picornavirus is *Enterovirus 71* (EV71) or *Coxsackievirus A14.*

3. The use according to claim 1 or 2, wherein the picornavirus is an aphthovirus;

   preferably, the aphthovirus is a foot-and-mouth disease virus;
   preferably, the foot-and-mouth disease virus is selected from the group consisting of the European serotypes (Serotype A, Serotype O, Serotype C), Asian serotype (Asian Serotype 1), and African serotypes (Serotype SAT1, Serotype SAT2, Serotype SAT3).

4. The use according to any one of claims 1 to 3, wherein the 3C protease has an amino acid sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the amino acid sequence as set forth in SEQ ID NO: 1;
   preferably, the 3C protease has an amino acid sequence as set forth in SEQ ID NO: 1.

5. The use according to any one of claims 1 to 4, wherein the nucleic acid molecule is a DNA molecule or an RNA molecule.

6. The use according to claim 5, wherein the nucleic acid molecule is an RNA molecule;

   preferably, the nucleic acid molecule comprises a coding sequence of the 3C protease;
   preferably, the nucleic acid molecule further comprises one or more selected from the group consisting of 5'UTR, Kozak sequence, initiation codon, termination codon, 3'UTR, and poly-A tail;
   preferably, the nucleic acid molecule comprises, from the 5' end to the 3' end in order: a 5'UTR, a Kozak sequence, an initiation codon, a coding sequence of the 3C protease, a termination codon, a 3'UTR, and a poly-A tail.

7. The use according to claim 6, which has one or more characteristics selected from the following:

   (1) the coding sequence of the 3C protease is codon-optimized according to the codon bias of the host cell (e.g., human cell) or not optimized;
   (2) the coding sequence of the 3C protease has a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the sequence as set forth in SEQ ID NO: 3; preferably, the coding sequence of the 3C protease is as set forth in SEQ ID NO: 3;
   (3) the nucleic acid molecule carries a 5' cap modification (e.g., Cap 0, Cap 1, Cap 2) at its 5' end; preferably, the nucleic acid molecule carries a 5' Cap 1 modification at its 5' end;
   (4) the nucleic acid molecule comprises one or more N1-methyl pseudouridine (m1$\psi$) modifications; preferably, some or all the uracil nucleotides in the isolated nucleic acid molecule are replaced with N1-methyl pseudouracil nucleotides;

(5) the coding sequence of the 3C protease carries one or more termination codons at its 3' end;

(6) the 5' UTR is selected from the group consisting of 5' UTR derived from human α-globin mRNA;

(7) the 3' UTR is selected from the group consisting of 3' UTR based on mtRNR1 (mitochondrial 12S ribosomal RNA) and AES (amino-terminal enhancer of split);

(8) the 5'UTR has a nucleotide sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 4; preferably, the 5'UTR has a nucleotide sequence as set forth in SEQ ID NO: 4;

(9) the 3'UTR has a nucleotide sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 6; preferably, the 3'UTR has a nucleotide sequence as set forth in SEQ ID NO: 6;

(10) the Kozak sequence is as set forth in SEQ ID NO: 5;

(11) the poly-A tail comprises one or more polyadenylic acid sequences, and the one or more polyadenylic acid sequences are each independently composed of 20 to 120 consecutive adenylic acids; preferably, the poly-A tail comprises multiple polyadenylic acid sequences, and the adjacent polyadenylic acid sequences are connected by a spacer sequence comprising non-A;

(12) the nucleic acid molecule has a nucleotide sequence having a sequence identity of at least 30%, at least 35%, at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% as compared with the nucleotide sequence as set forth in SEQ ID NO: 2; preferably, the nucleic acid molecule has a nucleotide sequence as set forth in SEQ ID NO: 2.

8. The use according to any one of claims 1 to 7, wherein the medicament is for use in the subject to:

(1) inhibit tumor cell proliferation;

(2) inhibit tumor cell migration and/or invasion;

(3) promote tumor cell apoptosis;

(4) downregulate the upstream p38 protein, downstream PSF protein and/or heterogeneous nuclear ribonucleoprotein (hnRNP) A1 in the MNK1/2 signaling pathway in tumor cell;

(5) upregulate the apoptosis execution protein caspase-3 in tumor cell; and/or

(6) inhibit the viability of tumor cell.

9. The use according to any one of claims 1 to 8, wherein the tumor is selected from the group consisting of solid tumors or hematological tumors (e.g., leukemia, lymphoma, myeloma);

preferably, the tumor is selected from the group consisting of glioma (e.g., glioblastoma), liver cancer, kidney cancer, melanoma, prostate cancer, colon cancer, cervical cancer, gastric cancer, lung cancer, colorectal cancer, bladder cancer, breast cancer, uterine/cervical cancer, ovarian cancer, gastrointestinal cancer, pancreatic cancer, skin cancer, lymphoma, leukemia, and sarcoma (e.g., osteosarcoma).

10. An isolated nucleic acid molecule, comprising a nucleotide sequence encoding a 3C protease, wherein the 3C protease is as defined in any one of claims 1 to 4;

preferably, the isolated nucleic acid molecule is the nucleic acid molecule as defined in any one of claims 5 to 7, or the isolated nucleic acid molecule encodes the 3C proteas as defined in any one of claims 5 to 7.

11. A vector, comprising the isolated nucleic acid molecule according to claim 10.

12. A delivery composition, comprising a delivery vehicle, and one or more selected from the group consisting of: a 3C protease, the isolated nucleic acid molecule according to claim 10, the vector according to claim 11;

wherein, the 3C protease is as defined in any one of claims 1 to 4;

preferably, the delivery vehicle is a particle;

preferably, the delivery vehicle is selected from the group consisting of lipid particle, sugar particle, metal particle, protein particle, liposome, exosome, microbubble, gene gun and viral vector (e.g., replication-defective retrovirus, lentivirus, adenovirus or adeno-associated virus).

13. The delivery composition according to claim 12, wherein the delivery vehicle is a lipid nanoparticle; preferably, the lipid nanoparticle comprises an ionizable cationic lipid SM-102, a helper lipid DSPC, a cholesterol and a PEGylated lipid PEG2000-DMG.

14. A pharmaceutical composition, comprising a 3C protease, the isolated nucleic acid molecule according to claim 10, the vector according to claim 11 and/or the delivery composition according to claim 12 or 13, and a pharmaceutically acceptable carrier and/or excipient;

   wherein, the 3C protease is as defined in any one of claims 1 to 4;
   preferably, the pharmaceutical composition further comprises an additional pharmaceutically active agent; preferably, the additional pharmaceutically active agent is a drug with anti-tumor activity; preferably, the additional pharmaceutically active agent is selected from the group consisting of: EGFR inhibitor, HER2 inhibitor, HER3 inhibitor, HER4 inhibitor, IGFR-1 inhibitor, mTOR inhibitor, PI3 kinase inhibitor, c-MET or VEGF inhibitor, chemotherapeutic drug, antibody drug, biomacromolecule drug, cellular therapeutic agent and any combination thereof;
   preferably, the additional pharmaceutically active agent and the 3C protease, the isolated nucleic acid molecule, the vector and/or the delivery composition are provided as independent components or mixed components.

15. A method for inhibiting a tumor cell, comprising: contacting a 3C protease, the isolated nucleic acid molecule according to claim 10, the vector according to claim 11, the delivery composition according to claim 12 or 13, or the pharmaceutical composition according to claim 14 with the tumor cell, or delivering the 3C protease, the isolated nucleic acid molecule, the vector, the delivery composition, or the pharmaceutical composition into the tumor cell; wherein, the 3C protease is as defined in any one of claims 1 to 4.

16. The method according to claim 15, wherein the method is for use to:

   (1) inhibit tumor cell proliferation;
   (2) inhibit tumor cell migration and/or invasion;
   (3) promote tumor cell apoptosis;
   (4) downregulate the upstream p38 protein, downstream PSF protein and/or heterogeneous nuclear ribonucleoprotein (hnRNP) A1 in the MNK1/2 signaling pathway in tumor cell;
   (5) upregulate the apoptosis execution protein caspase-3 in tumor cell; and/or
   (6) inhibit the viability of tumor cell.

17. The method according to claim 15 or 16, wherein the tumor is selected from the group consisting of solid tumors or hematological tumors (e.g., leukemia, lymphoma, myeloma); preferably, the tumor is selected from the group consisting of glioma (e.g., glioblastoma), liver cancer, kidney cancer, melanoma, prostate cancer, colon cancer, cervical cancer, gastric cancer, lung cancer, colorectal cancer, bladder cancer, breast cancer, uterine/cervical cancer, ovarian cancer, gastrointestinal cancer, pancreatic cancer, skin cancer, leukemia, and sarcoma (e.g., osteosarcoma).

18. Use of the isolated nucleic acid molecule according to claim 10, the vector according to claim 11, the delivery composition according to claim 12 or 13, or the pharmaceutical composition according to claim 14 in the preparation of a medicament for anti-tumor, or use of a 3C protease, the isolated nucleic acid molecule according to claim 10, the vector according to claim 11, the delivery composition according to claim 12 or 13, or the pharmaceutical composition according to claim 14 in the inhibition of a tumor cell, wherein the 3C protease is as defined in any one of claims 1 to 4.

A

EV71-pre3C IVT mRNA

T7 promoter

5' UTR

Kozak

Start codon

Protein coding region

stop codons

3' UTR

Segmented polyA

**SapI restriction enzyme site**

TAATACGACTCACTATAGGGAATAAACTAGTATTCTTCTGGTCCCCACAGACTCAGAGAGAACCCGC
**CACCAT**GGGCCCCAGCCTGGACTTCGCCCTGAGCCTGCTGCGGAGAAATATCCGGCAGGTGCAGACC
GATCAAGGCCACTTTACAATGCTGGGCGTGAGAGACCGGCTCGCCGTGCTGCCTCGCCACTCCCAGC
CTGGAAAGACCATTTGGATCGAGCATAAGCTGGTGAACGTGCTGGATGCCGTGGAACTGGTCGACGA
GCAGGGCGTGAACCTGGAGCTGACACTGATCACCCTGGACACCAACGAGAAGTTCCGGGACATCACC
AAATTCATCCCCGAAAACATCAGCACCGCCTCCGATGCTACACTGGTGATCAACACCGAGCACATGC
CTAGCATGTTCGTGCCCGTGGGCGACGTGGTGCAGTACGGCTTCCTGAACCTGAGCGGCAAGCCAAC
CCACCGGACAATGATGTACAATTTCCCTACAAAGGCCGGACAGTGCGGCGGGGTGGTGACCTCTGTG
GGCAAGGTGATCGGCATCCACATCGGCGGCAACGGAAGGCAGGGCTTTTGTGCCGGCCTGAAGAGAA
GCTACTTCGCCAGCGAGCAG**TGATGA**CTCGAGCTGGTACTGCATGCACGCAATGCTAGCTGCCCCTT
TCCCGTCCTGGGTACCCCGAGTCTCCCCCGACCTCGGGTCCCAGGTATGCTCCCACCTCCACCTGCC
CCACTCACCACCTCTGCTAGTTCCAGACACCTCCCAAGCACGCAGCAATGCAGCTCAAAACGCTTAG
CCTAGCCACACCCCCACGGGAAACAGCAGTGATTAACCTTTAGCAATAAACGAAAGTTTAACTAAGC
TATACTAACCCCAGGGTTGGTCAATTTCGTGCCAGCCACACCCTGGAGCTAGCAAAAAAAAAAAAAA
AAAAAAAAAAAAAAAAAGCATATGACTAAAAAAAAAAAAAAAAAAAAAAAAAAAAAAAAAAAAAAAA
AAAAAAAAAAAAAAAAAAAAAAAAAAAAAT**GAAGAGC**

B

FIG.1

FIG.2

FIG.3
(begining)

FIG.3 (end)

40

A

| | EV-3C | Cleaved caspased-3 | DAPI | Merge |

Control

EV-3C

B

| | EV-3C | Cleaved caspased-3 | DAPI | Merge |

Control

EV-3C

## FIG.4

**FIG.5**

**A**

**B**

FIG.6

FIG.7

**FIG.8**

A

Luciferase

5'UTR            3'UTR     Poly A

B

|         | 6 h.p.i | 24 h.p.i |
|---------|---------|----------|

i.v.

i.n.

C

i.v.

6 h.p.i

spleen          liver

FIG.9

**FIG.10**

**FIG.11**

**FIG.12**

| Cell line | $IC_{50}$ (ng/ml) | Therapeutic Index (TI) |
|---|---|---|
| HUVEC (human umbilical vein endothelial cell) | > 10000 | - |
| Huh7 (liver cancer) | 286.29±48.29 | > 24.99 |
| ACHN (human renal cell adenocarcinoma) | 287.33±94.13 | > 34.80 |
| U2OS (human osteosarcoma) | 271.97±10.42 | > 36.77 |
| CHL-1 (melanoma) | 362.42±70.88 | > 27.59 |
| DU145 (prostate cancer) | 319.28±85.93 | > 31.32 |
| HCT-116 (human colon cancer) | 649.29±116.53 | > 15.40 |
| Hela (cervical cancer) | 50.52±11.54 | > 199 |
| HepG2 (liver cancer) | 400±96.88 | > 24.99 |
| AGS (gastric cancer) | 126.34±48.68 | > 79.15 |
| A549 (lung cancer) | 221.99±18.24 | > 45.05 |
| MCF-7 (breast cancer) | 654.3±53.35 | > 15.28 |

# FIG.13

## INTERNATIONAL SEARCH REPORT

| International application No. |
| --- |
| **PCT/CN2024/116936** |

**A. CLASSIFICATION OF SUBJECT MATTER**

A61K38/48(2006.01)i; C12N15/57(2006.01)i; C12N15/85(2006.01)i; C12N15/86(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:A61K,C12N,A61P

Documentation searched other than minimum documentation to the extent that such documents are included in the fields searched

Electronic data base consulted during the international search (name of data base and, where practicable, search terms used)

CNTXT, ENTXT, VCN, DWPI, VEN, 超星读秀学术, 中国专利生物序列检索系统, CNKI, 万方, ISI web of knowledge, Elsevier Science Direct, Springerlink, pubmed, genbank, ENA, EMBL, Uniprot, STN, 百度, 百度学术, patentics: 中国人民解放军军事科学院军事医学研究院, ACADEMY OF MILITARY MEDICAL SCIENCES, 钟武, ZHONG Wu, 曹瑞源, CAO Ruiyuan, 杨晓童, YANG Xiaotong, 李微, LI Wei, 李松, LI Song, 小RNA病毒, 3C 蛋白酶, 3C protease, 3Cpro, EV71, 肠道病毒, enterovirus, 脊髓灰质炎病毒, 柯萨奇病毒, 鼻病毒, 埃可病毒, 口蹄疫病毒, 肿瘤, tumor, 癌, cancer, 胶质母细胞瘤, U87MG, A172, mRNA疫苗, 纳米粒子, SM-102, DSPC, 胆固醇, PEG2000-DMG, SEQ ID NOs: 1-7

**C. DOCUMENTS CONSIDERED TO BE RELEVANT**

| Category* | Citation of document, with indication, where appropriate, of the relevant passages | Relevant to claim No. |
| --- | --- | --- |
| X | LI.M.L. et al. "EV71 3C Protease Induces Apoptosis by Cleavage of hnRNP A1 to Promote Apaf-1 Translation" *PLOS ONE*, Vol. 14, No. 9, 09 September 2019 (2019-09-09), Article number: e0221048 (page 1, abstract, page 2, paragraph 5, and pages 4-13, Results) | 1-9, 14-18 |
| Y | LI.M.L. et al. "EV71 3C Protease Induces Apoptosis by Cleavage of hnRNP A1 to Promote Apaf-1 Translation" *PLOS ONE*, Vol. 14, No. 9, 09 September 2019 (2019-09-09), Article number: e0221048 (page 1, abstract, page 2, paragraph 5, and pages 4-13, Results) | 13-18 |
| X | LI,M.L. et al. "The 3C Protease Activity of Enterovirus 71 Induces Human Neural Cell Apoptosis" *Virology*, Vol. 293, No. 2,, 15 February 2002 (2002-02-15), page 386, abstract, and page 387, left-hand column, paragraph 4 to page 391, left-hand column, paragraph 1 | 1-9, 14-18 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **12 December 2024** | **23 January 2025** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/116936**

**C.    DOCUMENTS CONSIDERED TO BE RELEVANT**

| Category* | Citation of document, with indication, where appropriate, of the relevant passages | Relevant to claim No. |
|---|---|---|
| Y | LI,M.L. et al. "The 3C Protease Activity of Enterovirus 71 Induces Human Neural Cell Apoptosis"<br>*Virology*, Vol. 293, No. 2,, 15 February 2002 (2002-02-15),<br>    page 386, abstract, and page 387, left-hand column, paragraph 4 to page 391, left-hand column, paragraph 1 | 13-18 |
| X | CHAU,D.H.W. et al. "Coxsackievirus B3 Proteases 2A and 3C Induce Apoptotic Cell Death through Mitochondrial Injury and Cleavage of eIF4GI but not DAP5/p97/NAT1"<br>*Apoptosis*, Vol. 12, 29 December 2006 (2006-12-29),<br>    page 513, abstract, page 514, right-hand column, paragraph 2, and page 516, left-hand column, paragraph 2 from the bottom to page 520, left-hand column, paragraph 2 | 1-9, 14-18 |
| Y | CHAU,D.H.W. et al. "Coxsackievirus B3 Proteases 2A and 3C Induce Apoptotic Cell Death through Mitochondrial Injury and Cleavage of eIF4GI but not DAP5/p97/NAT1"<br>*Apoptosis*, Vol. 12, 29 December 2006 (2006-12-29),<br>    page 513, abstract, page 514, right-hand column, paragraph 2, and page 516, left-hand column, paragraph 2 from the bottom to page 520, left-hand column, paragraph 2 | 13-18 |
| X | LOTZERICH,M. et al. "Rhinovirus 3C Protease Suppresses Apoptosis and Triggers Caspase-Independent Cell Death"<br>*Cell Death and Disease*, Vol. vol. 9, 15 February 2018 (2018-02-15),<br>    Article number: 272 (page 1, abstract, and page 2, right-hand column, paragraph to page 12, left-hand column, paragraph 1) | 1-9, 14-18 |
| Y | LOTZERICH,M. et al. "Rhinovirus 3C Protease Suppresses Apoptosis and Triggers Caspase-Independent Cell Death"<br>*Cell Death and Disease*, Vol. vol. 9, 15 February 2018 (2018-02-15),<br>    Article number: 272 (page 1, abstract, and page 2, right-hand column, paragraph to page 12, left-hand column, paragraph 1) | 13-18 |
| X | BARCO,A. et al. "Poliovirus Protease 3C(pro) Kills Cells by Apoptosis"<br>*Virology*, Vol. 266, No. 2,, 20 January 2000 (2000-01-20),<br>    page 352, abstract, and page 353, left-hand column, paragraph 2 to page 356, right-hand column, paragraph 1 | 1-9, 14-18 |
| Y | BARCO,A. et al. "Poliovirus Protease 3C(pro) Kills Cells by Apoptosis"<br>*Virology*, Vol. 266, No. 2,, 20 January 2000 (2000-01-20),<br>    page 352, abstract, and page 353, left-hand column, paragraph 2 to page 356, right-hand column, paragraph 1 | 13-18 |
| X | US 2017056491 A1 (NATIONAL HEALTH RESEARCH INSTITUTES et al.) 02 March 2017 (2017-03-02)<br>    abstract, claims 10-14, description, paragraphs 12-13, 62-67 and 135, and sequence listing, SEQ ID NO: 5 | 10-12, 14 |
| Y | US 2017056491 A1 (NATIONAL HEALTH RESEARCH INSTITUTES et al.) 02 March 2017 (2017-03-02)<br>    abstract, claims 10-14, description, paragraphs 12-13, 62-67 and 135, and sequence listing, SEQ ID NO: 5 | 13-18 |
| X | CN 102199586 A (INSTITUTE OF MICROBIOLOGY, CHINESE ACADEMY OF SCIENCES) 28 September 2011 (2011-09-28)<br>    claim 1, description, paragraph 25, and sequence listing, SEQ ID NO: 1 | 10-12 |
| Y | CN 102199586 A (INSTITUTE OF MICROBIOLOGY, CHINESE ACADEMY OF SCIENCES) 28 September 2011 (2011-09-28)<br>    claim 1, description, paragraph 25, and sequence listing, SEQ ID NO: 1 | 13-18 |
| Y | CN 115590836 A (ZHEJIANG UNIVERSITY) 13 January 2023 (2023-01-13)<br>    abstract, and claims 1-10 | 13-18 |

Form PCT/ISA/210 (second sheet) (July 2022)

## INTERNATIONAL SEARCH REPORT

| International application No. |
| --- |
| **PCT/CN2024/116936** |

| **Box No. I** | **Nucleotide and/or amino acid sequence(s) (Continuation of item 1.c of the first sheet)** |
| --- | --- |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT

| International application No. |
| --- |
| **PCT/CN2024/116936** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **15-17**
   because they relate to subject matter not required to be searched by this Authority, namely:

   The subject matter of claims 15-17 relates to a tumor treatment method, and therefore falls within subject matter for which no search is required by the International Searching Authority as defined in PCT Rule 39.1(iv). Nevertheless, a search is still carried out on the basis of the pharmaceutical use of the 3C protease.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/116936**

| Patent document cited in search report | | | Publication date (day/month/year) | Patent family member(s) | | | Publication date (day/month/year) |
|---|---|---|---|---|---|---|---|
| US | 2017056491 | A1 | 02 March 2017 | KR | 20170068410 | A | 19 June 2017 |
| | | | | WO | 2015167710 | A1 | 05 November 2015 |
| | | | | SG | 11201609062 | PA | 29 November 2016 |
| | | | | TW | 201602347 | A | 16 January 2016 |
| | | | | TWI | 686475 | B | 01 March 2020 |
| | | | | US | 10004796 | B2 | 26 June 2018 |
| CN | 102199586 | A | 28 September 2011 | CN | 102199586 | B | 17 April 2013 |
| CN | 115590836 | A | 13 January 2023 | CN | 115590836 | B | 21 June 2024 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311152705

**Non-patent literature cited in the description**

- **HYYPIÄ T** ; **HOVI T** ; **KNOWLES NJ** ; **STANWAY G**. Classification of enteroviruses based on molecular and biological properties. *J Gen Virol*, 1997, vol. 78, 1-11
- **CONRAD L et al.** NCBI Taxonomy: a comprehensive update on curation, resources and tools. *Database: the journal of biological databases and curation*, 2020, vol. 2020, baaa062
- **NEEDLEMAN et al.** *J. Mol. Biol.*, 1970, vol. 48, 443-453
- **E. MEYERS** ; **W. MILLER**. *Comput. Appl. Biosci*, 1988, vol. 4, 11-17
- **NEEDLEMAN** ; **WUNSCH**. *J. Mol. Biol.*, 1970, vol. 48, 444-453
- **ORLANDINI VON NIESSEN AG et al.** Improving mRNA-Based Therapeutic Gene Delivery by Expression-Augmenting 3' UTRs Identified by Cellular Library Screening. *Mol Ther*, 10 April 2019, vol. 27 (4), 824-836
- Immunology-A Synthesis. Sinauer Associates, Sunderland, Mass., 1991
- Remington's Pharmaceutical Sciences. Mack Publishing Company, 1995
- **J. SAMBROOK**. Molecular Cloning: A Laboratory Manual. Cold Spring Harbor Laboratory Press, 1989
- **F. M. AUSUBEL**. Short Protocols in Molecular Biology. John Wiley & Sons, Inc